# EUROPEAN PATENT SPECIFICATION

(11) **EP 3 352 726 B1**
(45) Date of publication and mention of the grant of the patent: **25.12.2019**
(21) Application number: 16770289.3
(22) Date of filing: 23.09.2016
(51) Int. Cl.: A61K 8/25, A61K 8/58, A61K 8/891, A61K 8/26, A61K 8/31, A61Q 1/04, A61K 8/92, A61K 8/894

(54) **LIQUID COSMETIC COMPOSITION COMPRISING A HIGH NON-VOLATILE OIL CONTENT AND LIP TREATMENT PROCESS**
KOSMETISCHE FLÜSSIGKEITSZUSAMMENSETZUNG MIT HOHER GEHALT AN NICHTFLÜCHTIGEM ÖL UND VERFAHREN ZUR LIPPENPFLEGE
COMPOSITION COSMÉTIQUE LIQUIDE COMPRENANT UNE FORTE TENEUR EN HUILE NON VOLATILE, ET PROCÉDÉ DE TRAITEMENT DES LÈVRES

(30) Priority: 25.09.2015 FR 1559090
(43) Date of publication of application: 01.08.2018
(73) Proprietor: L'OREAL, 75008 Paris (FR)
(72) Inventor: ARDITTY, Stéphane, 94152 Chevilly Larue (FR); PRUD'HOMME, Estelle, 94152 Chevilly Larue (FR); JACQUES, Véronique, 94152 Chevilly Larue (FR); LE PADELLEC, Sylvie, 94152 Chevilly Larue (FR)
(74) Representative: L'Oreal
(86) International application number: PCT/EP2016/072738
(87) International publication number: WO 2017/050992

(56) References cited:
- EP-A1- 2 206 492
- WO-A1-2013/102065
- FR-A1- 2 992 193
- FR-A1- 2 992 215

## Description

The subject of the present invention is a composition intended more particularly for making up and/or caring for the lips, which is in a liquid form and which comprises at least 70% by weight of non-volatile oils, at least 1% by weight of at least one non-ionic silicone surfactant, and coloured or non-coloured solid particles. The present invention also relates to a process using said composition, consisting in applying it to the lips.

Formulas intended for making up and/or caring for the lips have been known for a very long time and are in varied forms, ranging from fluid formulas such as glosses, to optionally supported solid compositions in the form of a stick, or even compositions in the form of a pencil.

In order to further diversify the compositions provided to consumers, anhydrous or virtually anhydrous formulas, which are even more fluid than the glosses, and which comprise high contents of oils, that are very comfortable to wear, have emerged. However, these formulas are available only in versions that are not very coloured. This comes essentially from the fact that it is very difficult to maintain high contents of particles in suspension in this type of oily medium, without losing the attraction of such compositions, which is precisely their great fluidity.

Indeed, with large amounts of pigments, nacres or fillers, sedimentation of these particles is observed, resulting in the formation of a relatively unattractive cake which can comprise clumps of particles. The cake thus formed has the additional drawback of being very cohesive. Thus, it cannot be easily redispersed so as to again have a uniform composition. Consequently, once the particles have sedimented, the composition becomes virtually unusable and no longer makes it possible to obtain a uniform deposit on the lips, which is not acceptable.

Thus, in order to minimize the sedimentation problem, these compositions comprise relatively low contents of solid particles. However, in this case, the ranges of shades and the colour results (in terms of coverage for example) that are accessible are limited. The compositions can also comprise dyes, therefore species that are soluble in the medium in which they are used. However, the number of dyes that can be used in this field is relatively restricted, which does not allow a great deal of extension of the range of colours proposed or of their intensity, whether or not the dyes are combined with coloured solid particles.

It is therefore sought to obtain compositions intended for making up and/or caring for the lips, which are very fluid and which comprise high contents of non-volatile oils, which are easy to use and the storage of which does not pose any difficulties in terms of re-use of the product.

These aims and others are achieved by the present invention, a subject of which is thus liquid cosmetic compositions comprising:
* at least 70% by weight, relative to the weight of the composition, of at least one non-volatile oil or of a single-phase mixture of several non-volatile oils, the composition comprising at least one polar or non-polar, non-volatile hydrocarbon-based oil;
* at least one mineral thickener;
* at least one non-ionic silicone surfactant in a content of at least 1% by weight, relative to the weight of the composition;
* at least coloured or non-coloured solid particles.

A subject of the invention is also a process for making up and/or caring for the lips, consisting in applying such a composition.

One of the advantages of the composition according to the invention is that, if particle sedimentation is observed, this phenomenon, which previously posed very significant problems, is converted in the present case into an asset.

Specifically, when the solid particles present in the composition according to the invention sediment when said composition is left to stand, they allow the appearance of a translucent-to-clear supernatant liquid phase which is more or less coloured according to the pigments and optionally the dyes present, and a sedimentary particulate phase, the appearance of which is uniform, without clumps (lumps).

The composition therefore retains an attractive and aesthetic visual appearance, thereby allowing packaging in a transparent bottle.

Another considerable advantage of the composition according to the invention is that it is enough to simply shake the bottle to re-suspend the cake thus formed, and to return to a fluid and uniform composition, which is easily applicable and which leaves a uniform, glossy, comfortable, non-tacky film.

The composition also makes it possible to obtain a stronger colouration deposit, in more varied shades than those possible with existing compositions of this type.

Other advantages of the present invention will emerge more clearly on reading the description and the examples that follow.

In what follows, the wording "comprising a" or "containing a" means "comprising at least one" or "containing at least one", unless otherwise specified. The expressions "at least one" and "several" are used without distinction.

Furthermore, unless otherwise indicated, the limits indicated for a range are included in that range.

The composition according to the invention is in the form of a liquid at ambient temperature (25°C) and atmospheric pressure (1.013×10⁵ Pa).

More particularly, the viscosity of the composition varies between 0.2 and 0.8 Pa.s, preferably between 0.3 and 0.7 Pa.s.

The viscosity measurement is generally performed at 25°C, using a Rheomat RM180 viscometer equipped with a No. 2 spindle, the measurement being performed after 10 minutes of rotation of the spindle in the composition (after which time stabilization of the viscosity and of the spin speed of the spindle are observed), at 200 rpm.

As previously indicated, according to one particularly advantageous embodiment of the invention, the composition sediments when it is left to stand.

The composition is said to sediment when it allows the appearance of a supernatant liquid after standing for 48 hours at 25°C, measured under the following conditions:
The measurements are carried out in a cylindrical polyethylene terephthalate receptacle, the height of which is 46.9 mm and the radius of which is 15 mm, comprising two steel balls 6.35 mm in diameter, and fitted with a stopper.

The composition is introduced such that the height in the bottle reaches 3.5 cm (which represents approximately 4.5 g of composition) and the bottle is stoppered.

Said bottle is left for 48 hours at a temperature of 25°C.

After the 48 hours, the height of supernatant liquid is measured.

It is considered that the composition sediments when the height of supernatant liquid represents at least 5% of the total height of the composition, preferably at least 15% of the total height of the composition.

### NON-VOLATILE OILS

As previously indicated, the composition according to the invention comprises at least 70% by weight, relative to the weight of the composition, of at least one non-volatile oil or of a single-phase mixture of several non-volatile oils.

The mixture is said to be single-phase when no phase separation is observed by eye or under a phase-contrast microscope, at ambient temperature (25°C) after homogenization at temperature and mixing on a Rayneri mixer (550 rpm, 10 minutes) and storage while left to stand in a closed receptacle at ambient temperature for 24 hours (and atmospheric pressure). Under these conditions, the term stable single-phase mixture is used.

As previously indicated, the composition comprises one or more non-volatile oils chosen from polar or non-polar hydrocarbon-based oils.

More particularly, for the purposes of the invention, the word "oil" denotes non-aqueous compounds that are liquid at 25°C and atmospheric pressure (1.013 ×.10⁵ Pa), and water-immiscible. The term "immiscible" is intended to mean that the mixing of the same amount of water and oil, after mixing (for example Rayneri 550 rpm; 10 minutes), does not result in a stable solution comprising just one phase, under normal temperature and pressure conditions.

The term "non-volatile oil" is intended to mean an oil of which the vapour pressure at 25°C and atmospheric pressure is non-zero and is less than 0.02 mmHg (2.66 Pa) and better still less than 10⁻³ mmHg (0.13 Pa).

The term "hydrocarbon-based oil" is intended to mean an oil formed essentially from, or even constituted by, carbon and hydrogen atoms, and optionally oxygen and nitrogen atoms, and not containing any silicon or fluorine atoms.

For the purposes of the present invention, the term "non-polar oil" is intended to mean an oil chosen from hydrocarbons, i.e. from compounds comprising only carbon and hydrogen atoms.

### Non-polar non-volatile hydrocarbon-based oils

These oils may be of plant, mineral or synthetic origin.

More particularly, the non-polar non-volatile hydrocarbon-based oil(s) can be chosen from linear or branched hydrocarbons of mineral or synthetic origin.

For example, the following are suitable for carrying out the present invention:
- liquid paraffins,
- squalane,
- isohexadecane,
- isoeicosane,
- naphthalene oil,
- polybutenes, for instance Indopol H-100 (molar mass or MW = 965 g/mol), Indopol H-300 (MW = 1340 g/mol) and Indopol H-1500 (MW = 2160 g/mol) sold or manufactured by the company Amoco,
- polyisobutenes, hydrogenated polyisobutenes, for instance Parleam® sold by the company Nippon Oil Fats, Panalane H-300 E sold or manufactured by the company Amoco (MW = 1340 g/mol), Viseal 20000 sold or manufactured by the company Synteal (MW = 6000 g/mol) and Rewopal PIB 1000 sold or manufactured by the company Witco (MW = 1000 g/mol), or alternatively Parleam Lite sold by NOF Corporation,
- decene/butene copolymers and polybutene/polyisobutene copolymers, in particular Indopol L-14,
- polydecenes and hydrogenated polydecenes, for instance: Puresyn 10 (MW = 723 g/mol) and Puresyn 150 (MW = 9200 g/mol) sold or manufactured by the company Mobil Chemicals, or alternatively Puresyn 6 sold by ExxonMobil Chemical,
- and mixtures thereof.

Preferably, the non-polar non-volatile hydrocarbon-based oil(s) are chosen from hydrogenated or non-hydrogenated polybutenes, hydrogenated or non-hydrogenated polyisobutenes and hydrogenated or non-hydrogenated polydecenes, and also mixtures thereof.

### Polar non-volatile hydrocarbon-based oils

These polar hydrocarbon-based oils are thus formed from carbon and hydrogen atoms, and comprise at least one or more oxygen or nitrogen atoms, but are free of silicon or fluorine atoms.

They consequently contain at least one group chosen from alcohol, ester, ether, carboxylic acid, amine and/or amide functions.

Preferably, the polar non-volatile hydrocarbon-based oils are, in addition to being free of silicon and fluorine, free of heteroatoms such as nitrogen and phosphorus.

In the present case, the polar non-volatile hydrocarbon-based oils therefore comprise one or more oxygen atoms as heteroatom.

In particular, the polar non-volatile hydrocarbon-based oil(s) comprise at least one alcohol function (it is then an "alcohol oil") or at least one ester function (it is then an "ester oil"). It should be noted that the ester oils may in particular be hydroxylated.

The composition may comprise one or more non-volatile hydrocarbon-based oils, in particular chosen from:
- saturated or unsaturated, branched or non-branched C₁₀-C₂₆, in particular C₁₀-C₂₄ and preferably C₁₂-C₂₂ alcohols, more particularly monoalcohols.

Advantageously, the C₁₀-C₂₆ alcohols are fatty monoalcohols, which are preferably branched when they comprise at least 16 carbon atoms.

As examples of fatty alcohols that may be used according to the invention, mention may be made of linear or branched fatty alcohols, of synthetic origin or alternatively of natural origin, for instance alcohols derived from plant material (coconut, palm kernel, palm, etc.) or animal material (tallow, etc.).

Needless to say, other long-chain alcohols may also be used, for instance ether alcohols or alternatively "Guerbet" alcohols.

Finally, use may also be made of certain fractions of alcohols of varying length of natural origin, for instance coconut (C₁₂ to C₁₆) or tallow (C₁₆ to C₁₈) or compounds such as diols or cholesterol.

As particular examples of fatty alcohols that may preferably be used, mention may be made in particular of lauryl alcohol, isostearyl alcohol, oleyl alcohol, 2-butyloctanol, 2-undecylpentadecanol, 2-hexyldecyl alcohol, isocetyl alcohol and octyldodecanol, and mixtures thereof.

According to an advantageous embodiment of the invention, the alcohol is octyldodecanol;
- optionally hydroxylated monoesters, diesters or triesters of a C₂-C₈ monocarboxylic or polycarboxylic acid and of a C₂-C₈ alcohol.
   In particular:
   * optionally hydroxylated monoesters of a C₂-C₈ carboxylic acid and of a C₂-C₈ alcohol,
   * optionally hydroxylated diesters of a C₂-C₈ dicarboxylic acid and of a C₂-C₈ alcohol, such as, for example, diisopropyl adipate, 2-diethylhexyl adipate, dibutyl adipate or 2-diethylhexyl succinate,
   * optionally hydroxylated triesters of a C₂-C₈ tricarboxylic acid and of a C₂-C₈ alcohol, such as, for example, citric acid esters, such as trioctyl citrate, triethyl citrate, acetyl tributyl citrate, tributyl citrate or acetyl tributyl citrate;
- esters of a C₂-C₈ polyol and of one or more C₂-C₈ carboxylic acids, such as glycol diesters of monoacids, such as in particular neopentyl glycol diheptanoate, propylene glycol dioctanoate, or glycerol triesters of monoacids, such as triacetin;
- ester oils, in particular containing at least 18 carbon atoms and even more particularly between 18 and 70 carbon atoms.
   Examples that may be mentioned include monoesters, diesters or triesters.
   The ester oils may be hydroxylated or non-hydroxylated.
   Thus, the non-volatile ester oil may be chosen, for example, from:
   * monoesters comprising at least 18 carbon atoms and even more particularly comprising between 18 and 40 carbon atoms in total, in particular the monoesters of formula R₁COOR₂ in which R₁ represents a saturated or unsaturated, linear or branched or aromatic fatty acid residue comprising from 4 to 35 carbon atoms and R₂ represents a hydrocarbon-based chain, which is in particular branched, containing from 4 to 35 carbon atoms, on condition that the sum of the carbon atoms of the radicals R₁ and R₂ is greater than or equal to 18, for instance Purcellin oil (cetostearyl octanoate), isononyl isononanoate, 2-ethylhexyl palmitate, octyldodecyl neopentanoate, 2-octyldodecyl stearate, 2-octyldodecyl erucate, isostearyl isostearate, C₁₂-C₁₅ alkyl benzoates, such as 2-octyldodecyl benzoate, alcohol or polyalcohol octanoates, decanoates or ricinoleates, isopropyl myristate, isopropyl palmitate, butyl stearate, hexyl laurate, 2-ethylhexyl palmitate, 2-hexyldecyl laurate, 2-octyldecyl palmitate or 2-octyldodecyl myristate.
      Preferably, they are esters of formula R₁COOR₂ in which R₁ represents a linear or branched fatty acid residue comprising from 4 to 35 carbon atoms and R₂ represents a hydrocarbon-based chain that is in particular branched, containing from 4 to 35 carbon atoms, R₁ and R₂ being such that the sum of the carbon atoms of the radicals R₁ and R₂ is greater than or equal to 18;
   * monoesters, in particular containing at least 18 carbon atoms and even more particularly from 18 to 22 carbon atoms, of a fatty acid in particular such as lanolic acid, oleic acid, lauric acid or stearic acid, and of diols such as glycols, for instance propylene glycol monoisostearate;
   * diesters, in particular containing at least 18 carbon atoms and even more particularly comprising between 18 and 60 carbon atoms in total and in particular between 18 and 50 carbon atoms in total. Use may be made in particular of diesters of a dicarboxylic acid and of monoalcohols comprising more than 8 carbon atoms, preferably such as diisostearyl malate, diisostearyl adipate; or glycol diesters of monocarboxylic acids, such as, for example, neopentyl glycol diheptanoate, diethylene glycol diisononanoate; or polyglyceryl-2 diisostearate (in particular such as the compound sold under the commercial reference Dermol DGDIS by the company Akzo);
   * hydroxylated monoesters and diesters, preferably with a total carbon number of at least 18 carbon atoms and even more particularly ranging from 18 to 70, such as in particular polyglyceryl-3 diisostearate, isostearyl lactate, octyl hydroxystearate, octyldodecyl hydroxystearate, diisostearyl malate or glyceryl stearate;
   * triesters, in particular containing at least 35 carbon atoms and even more particularly comprising between 35 and 70 carbon atoms in total, in particular such as triesters of a tricarboxylic acid, such as, example, triisostearyl citrate, or tridecyl trimellitate, or glyceryl triesters of monocarboxylic acids such as polyglyceryl-2 triisostearate;
   * tetraesters, in particular containing at least 35 carbon atoms and even more particularly with a total carbon number ranging from 35 to 70, such as, for example, pentaerythritol or polyglycerol tetraesters of a monocarboxylic acid, for instance pentaerythrityl tetrapelargonate, pentaerythrityl tetraisostearate, pentaerythrityl tetraisononanoate, glyceryl tris(2-decyl)tetradecanoate, polyglyceryl-2 tetraisostearate or pentaerythrityl tetrakis(2-decyl)tetradecanoate;
   * polyesters obtained by condensation of an unsaturated fatty acid dimer and/or trimer and of diols such as those described in patent application FR 0 853 634. In particular, the unsaturated fatty acid dimer may comprise from 28 to 44 carbon atoms, 2 carboxylic acid functions and 2 to 4 unsaturations; the unsaturated fatty acid trimer may comprise from 42 to 66 carbon atoms, 3 carboxylic acid functions and also 3 to 6 unsaturations. Preferably, use is made of an unsaturated fatty acid dimer, in particular containing 36 carbon atoms and 2 carboxylic acid functions. Mixtures of unsaturated fatty acid dimers and trimers and/or of unsaturated fatty acid (not polymerized, thus corresponding to a monomer) may also be used. Moreover, the diol comprises from 2 to 10 carbon atoms and two hydroxyl functions. In particular, mention may be made of esters of dilinoleic acid and of 1,4-butanediol or propanediol. Mention may in particular be made in this respect of the polymer sold by Biosynthis under the name Viscoplast 14436H (INCI name: dilinoleic acid/butanediol copolymer), or else copolymers of polyols and of diacid dimers, and esters thereof, such as Hailucent ISDA;
   * esters and polyesters of diol dimer and of monocarboxylic or dicarboxylic acid, such as esters of diol dimer and of fatty acid and esters of diol dimer and of dicarboxylic acid dimer, in particular which may be obtained from a dicarboxylic acid dimer derived in particular from the dimerization of an unsaturated fatty acid in particular of C₈ to C₃₄, in particular of C₁₆ to C₂₀ and more particularly esters of dilinoleic diacids and of dilinoleic diol dimers, for example sold by the company Nippon Fine Chemical under the trade names Lusplan DD-DA5® and DD-DA7®;
   * polyesters resulting from the esterification of at least one triglyceride of hydroxylated carboxylic acid(s) with an aliphatic monocarboxylic acid and with an aliphatic dicarboxylic acid, which is optionally unsaturated, for instance the succinic acid and isostearic acid castor oil sold under the reference Zenigloss by Zenitech;
   * triglyceride oils of natural or synthetic origin, for instance hydrocarbon-based plant oils such as, for example, jojoba oil; and unsaturated triglycerides such as castor oil, olive oil, ximenia oil or pracaxi oil; fatty acid triglycerides (which are liquid at ambient temperature and atmospheric pressure), in particular of fatty acids, which are saturated or unsaturated, containing at least 7 carbon atoms and even more particularly containing from 7 to 40 carbon atoms, such as heptanoic or octanoic acid triglycerides or saturated triglycerides such as caprylic/capric acid triglyceride and mixtures thereof, for example such as the product sold under the reference Myritol 318 from Cognis, glyceryl triheptanoate, glyceryl trioctanoate, and C₁₈₋₃₆ acid triglycerides such as those sold under the reference Dub TGI 24 by Stéarineries Dubois;
- vinylpyrrolidone/1-hexadecene copolymers, for instance the product sold under the name Antaron V-216 (also known as Ganex V216) by the company ISP;
- C₁₂-C₂₆ fatty acids, preferably C₁₂-C₂₂ fatty acids, which are preferably unsaturated, such as oleic acid, linoleic acid or linolenic acid, and mixtures thereof;
- dialkyl carbonates, the 2 alkyl chains possibly being identical or different, such as dicaprylyl carbonate sold under the name Cetiol CC® by Cognis;
- and mixtures thereof.

In accordance with one particularly advantageous embodiment of the invention, the composition comprises at least one polar non-volatile hydrocarbon-based oil. Preferably, the polar non-volatile hydrocarbon-based oil(s) are chosen from ester oils, in particular hydroxylated or non-hydroxylated monoesters and diesters comprising at least 18 carbon atoms in total, triesters, in particular containing at least 35 carbon atoms, tetraesters, in particular containing at least 35 carbon atoms, and mixtures thereof.

### Non-volatile phenyl silicone oils

The composition according to the invention may optionally comprise at least one non-volatile phenyl silicone oil.

The expression "phenyl silicone oil" denotes a silicone oil bearing at least one phenyl substituent.

These non-volatile phenyl silicone oils may be chosen from those also bearing at least one dimethicone fragment, or from those not bearing any.

The term "dimethicone fragment" denotes a divalent siloxane group in which the silicon atom bears two methyl radicals, this group not being located at the ends of the molecule. It may be represented by the following formula: -(Si(CH₃)₂-O)-.

The non-volatile phenyl silicone oil may thus be chosen from:
**a)** phenyl silicone oils optionally bearing a dimethicone fragment corresponding to formula (I) below: 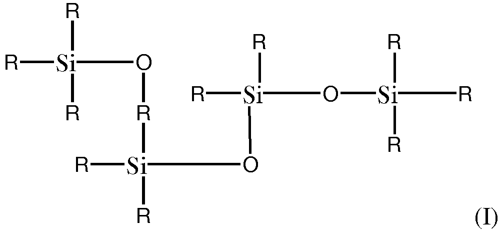 in which the groups R, which are monovalent or divalent, represent, independently of each other, a methyl, methylene, phenyl or phenylene, with the proviso that at least one group R represents a phenyl.
   More particularly, the phenyl silicone oil of formula (I) comprises at least three, for example at least four, advantageously at least five or at least six, phenyl groups;
**b)** phenyl silicone oils optionally bearing a dimethicone fragment corresponding to formula (II) below: 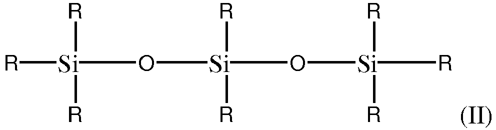 in which the groups R represent, independently of each other, a methyl or a phenyl, with the proviso that at least one group R represents a phenyl.
   Preferably, in this formula, the compound of formula (II) comprises at least three, for example at least four or at least five, phenyl groups.
   Mixtures of different phenyl silicone compounds described previously may be used.
   Among the compounds of formula (II), mention may particularly be made of phenyl silicone oils not bearing any dimethicone fragments with at least 4 or at least 5 radicals R representing a phenyl radical, the remaining radicals representing methyls.
   Such non-volatile phenyl silicone oils are preferably trimethylpentaphenyltrisiloxane or tetramethyltetraphenyltrisiloxane, for example PH-1555 HRI or Dow Corning 555 Cosmetic Fluid (INCI name: trimethyl pentaphenyltrisiloxane), and Dow Corning 554 Cosmetic Fluid by Dow Corning (INCI name: tetramethyl-tetraphenyl-trisiloxane), sold by the company Dow Corning.
   They correspond in particular to the following formulae (III), (III'): 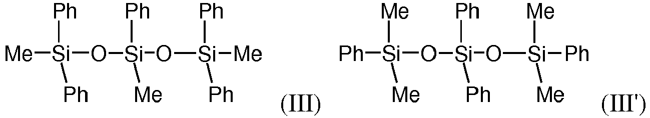 in which Me represents methyl, and Ph represents phenyl.
**c)** phenyl silicone oils bearing at least one dimethicone fragment corresponding to formula (IV) below: 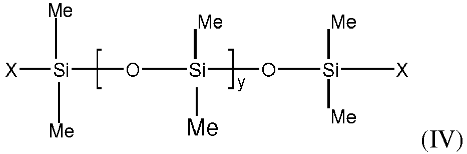 in which Me represents methyl, y is between 1 and 1000 and X represents -CH₂-CH(CH₃)(Ph).
**d)** phenyl silicone oils corresponding to formula (V) below, and mixtures thereof: 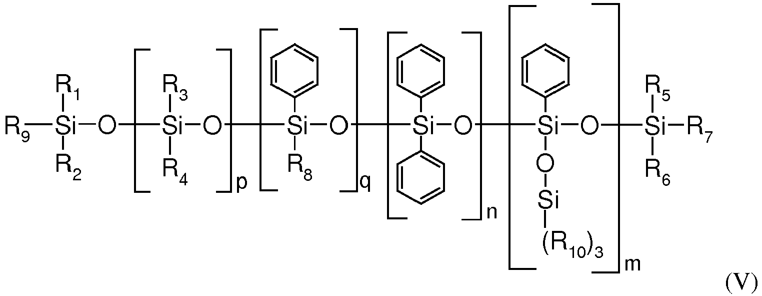 in which:
   - R₁ to R₁₀, independently of each other, are saturated or unsaturated, linear, cyclic or branched C₁-C₃₀ hydrocarbon-based radicals,
   - m, n, p and q are, independently of each other, integers between 0 and 900, with the proviso that the sum m+n+q is other than 0.

Preferably, the sum m+n+q is between 1 and 100. Advantageously, the sum m+n+p+q is between 1 and 900 and preferably between 1 and 800.

Preferably, q is equal to 0.

More particularly, R₁ to R₁₀, independently of each other, represent a saturated or unsaturated, preferably saturated, linear or branched C₁-C₃₀ hydrocarbon-based radical, and in particular a preferably saturated C₁-C₂₀, in particular C₁-C₁₈, hydrocarbon-based radical, or a monocyclic or polycyclic C₆-C₁₄, and in particular C₁₀-C₁₃, aryl radical, or an aralkyl radical, the alkyl part of which is preferably C₁-C₃ alkyl.

Preferably, R₁ to R₁₀ may each represent a methyl, ethyl, propyl, butyl, isopropyl, decyl, dodecyl or octadecyl radical, or alternatively a phenyl, tolyl, benzyl or phenethyl radical. R₁ to R₁₀ may in particular be identical, and in addition may be a methyl radical.

According to a first more particular embodiment of formula (V), mention may be made of:

### i) phenyl silicone oils optionally bearing at least one dimethicone fragment corresponding to formula (VI) below, and mixtures thereof:

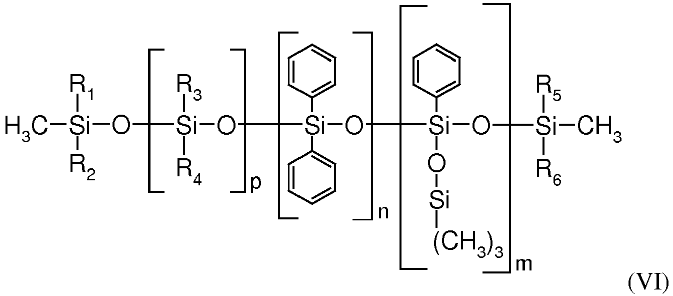

in which:
- R₁ to R₆, independently of each other, are saturated or unsaturated, linear, cyclic or branched C₁-C₃₀ hydrocarbon-based radicals, a preferably C₆-C₁₄ aryl radical or an aralkyl radical, the alkyl part of which is C₁-C₃ alkyl,
- m, n and p are, independently of each other, integers between 0 and 100, with the proviso that the sum n+m is between 1 and 100.

Preferably, R₁ to R₆, independently of each other, represent a C₁-C₂₀, in particular C₁-C₁₈, hydrocarbon-based, preferably alkyl, radical, or a C₆-C₁₄ aryl radical which is monocyclic (preferably C₆) or polycyclic and in particular C₁₀-C₁₃, or an aralkyl radical (preferably the aryl part is C₆ aryl; the alkyl part is C₁-C₃ alkyl).

Preferably, R₁ to R₆ may each represent a methyl, ethyl, propyl, butyl, isopropyl, decyl, dodecyl or octadecyl radical, or alternatively a phenyl, tolyl, benzyl or phenethyl radical.

R₁ to R₆ may in particular be identical, and in addition may be a methyl radical. Preferably, m = 1 or 2 or 3, and/or n = 0 and/or p = 0 or 1 may be applied, in formula (VI).

Preferably, use may be made of oils corresponding to compounds of formula (VI) in which:
**A)** m = 0 and n and p are, independently of each other, integers between 1 and 100.
   Preferably, R₁ to R₆ are methyl radicals.
   According to this embodiment, the silicone oil is preferably chosen from a diphenyl dimethicone such as KF-54 from Shin-Etsu (400 cSt), KF54HV from Shin-Etsu (5000 cSt), KF-50-300CS from Shin-Etsu (300 cSt), KF-53 from Shin-Etsu (175 cSt) or KF-50-100CS from Shin-Etsu (100 cSt).
**B)** p is between 1 and 100, the sum n+m is between 1 and 100, and n = 0.

These phenyl silicone oils optionally bearing at least one dimethicone fragment correspond more particularly to formula (VII) below: 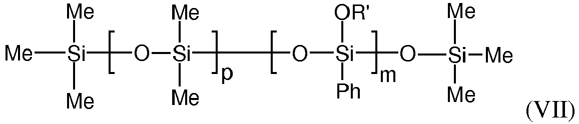 in which Me is methyl and Ph is phenyl, OR' represents a group -OSiMe₃ and p is 0 or is between 1 and 1000, and m is between 1 and 1000. In particular, m and p are such that compound (VII) is a non-volatile oil.

According to a first embodiment of non-volatile phenyl silicone bearing at least one dimethicone fragment, p is between 1 and 1000. m is more particularly such that the compound (VII) is a non-volatile oil. Use may be made, for example, of trimethylsiloxyphenyl dimethicone, sold in particular under the reference Belsil PDM 1000 by the company Wacker.

According to a second embodiment of non-volatile phenyl silicone not bearing a dimethicone fragment, p is equal to 0. m is between 1 and 1000, and in particular is such that the compound (VII) is a non-volatile oil.

Phenyltrimethylsiloxytrisiloxane, sold in particular under the reference Dow Corning 556 Cosmetic Grade Fluid (DC556) by the company Dow Corning, may, for example, be used.

### ii) non-volatile phenyl silicone oils not bearing a dimethicone fragment corresponding to formula (VIII) below, and mixtures thereof:

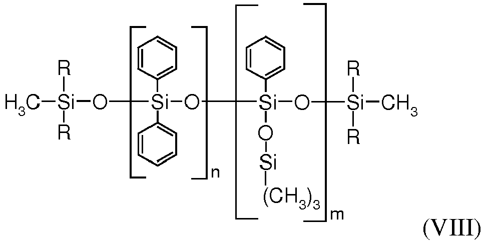

in which:
- R, independently of each other, are saturated or unsaturated, linear, cyclic or branched C₁-C₃₀ hydrocarbon-based radicals, preferably R is a C₁-C₃₀ alkyl radical, preferably a C₆-C₁₄ aryl radical, or an aralkyl radical, the alkyl part of which is C₁-C₃ alkyl,
- m and n are, independently of each other, integers between 0 and 100, with the proviso that the sum n+m is between 1 and 100.

Preferably, R, independently of each other, represent a saturated or unsaturated, preferably saturated, linear or branched C₁-C₃₀ hydrocarbon-based radical, and in particular a preferably saturated, C₁-C₂₀, in particular C₁-C₁₈ and more particularly C₄-C₁₀, hydrocarbon-based radical, a monocyclic or polycyclic C₆-C₁₄, and in particular C₁₀-C₁₃, aryl radical, or an aralkyl radical of which preferably the aryl part is C₆ aryl and the alkyl part is C₁-C₃ alkyl.

Preferably, the R may each represent a methyl, ethyl, propyl, butyl, isopropyl, decyl, dodecyl or octadecyl radical, or alternatively a phenyl, tolyl, benzyl or phenethyl radical.

The radicals R may in particular be identical, and in addition may be a methyl radical.

Preferably, m = 1 or 2 or 3, and/or n = 0 and/or p = 0 or 1 may be applied, in formula (VIII).

According to one preferred embodiment, n is an integer between 0 and 100 and m is an integer between 1 and 100, with the proviso that the sum n+m is between 1 and 100, in formula (VIII). Preferably, R is a methyl radical.

According to this embodiment, the non-volatile phenyl silicone oil is preferably chosen from phenyl trimethicones (when n=0) such as DC556 from Dow Corning, or else from diphenylsiloxyphenyl trimethicone oil (when m and n are between 1 and 100) such as KF56 A from Shin-Etsu, or the Silbione 70663V30 oil from Rhône-Poulenc.

### e) phenyl silicone oils optionally bearing at least one dimethicone fragment corresponding to the following formula, and mixtures thereof:

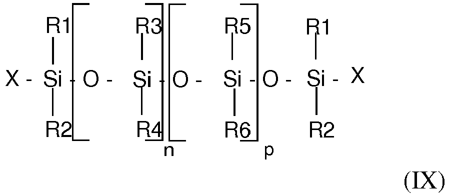

in which:
R₁, R₂, R₅ and R₆, which may be identical or different, are an alkyl radical containing 1 to 6 carbon atoms,
R₃ and R₄, which may be identical or different, are an alkyl radical containing from 1 to 6 carbon atoms or an aryl radical (preferably C₆-C₁₄), with the proviso that at least one of R₃ and R₄ is a phenyl radical,
X is an alkyl radical containing from 1 to 6 carbon atoms, a hydroxyl radical or a vinyl radical,
n and p are integers greater than or equal to 1, chosen so as to give the oil a weight-average molecular mass of less than 200 000 g/mol, preferably less than 150 000 g/mol and more preferably less than 100 000 g/mol.

### f) and a mixture thereof.

The composition according to the invention therefore comprises at least 70% by weight, relative to the weight of the composition, of a non-volatile oil or of a single-phase mixture of several non-volatile oils, at least one of which is a polar or non--polar, non-volatile hydrocarbon-based oil. More particularly, the content of non-volatile oil(s) represents from 70% to 90% by weight, more specifically from 75% to 90% by weight and preferably from 75% to 85% by weight relative to the weight of the composition.

If the composition comprises at least one non-volatile phenyl silicone oil, then its content preferably does not exceed 20% by weight, more advantageously does not exceed 15% by weight, and even more preferentially does not exceed 10% by weight, relative to the weight of the composition.

According to one particular embodiment of the invention, the composition does not comprise any non-volatile phenyl silicone oil.

It should be noted that, if the composition comprises a mixture of non-volatile oil(s), then said mixture is preferably single-phase. More particularly, the mixture is said to be single-phase when no phase separation is observed by eye or under a phase-contrast microscope, at ambient temperature (25°C) after homogenization at temperature and mixing on a Rayneri mixer (550 rpm, 10 minutes) and storage while left to stand in a closed receptacle at ambient temperature (and atmospheric pressure) for 24 hours.

Preferably, the composition according to the invention comprises at least one polar non-volatile hydrocarbon-based oil, more particularly chosen from ester oils, in particular hydroxylated or non-hydroxylated monoesters and diesters comprising at least 18 carbon atoms in total, triesters, in particular containing at least 35 carbon atoms, tetraesters, in particular containing at least 35 carbon atoms, and mixtures thereof.

The composition according to the invention preferably comprises, moreover, at least one non-polar non-volatile hydrocarbon-based oil, more particularly chosen from hydrogenated or non-hydrogenated polybutenes, hydrogenated or non-hydrogenated polyisobutenes, hydrogenated or non-hydrogenated polydecenes, and mixtures thereof.

In accordance with one particularly advantageous embodiment, the composition according to the invention comprises a mixture of polar and non-polar hydrocarbon-based oils.

Preferably, the weight proportion of polar non-volatile hydrocarbon-based oil(s) relative to the non-polar non-volatile hydrocarbon-based oil(s) is greater than or equal to 1, more advantageously greater than or equal to 2.

### MINERAL THICKENER

The composition according to the invention comprises at least one mineral thickener more particularly chosen from optionally modified organophilic clays, silicas, which are preferably hydrophobic, and mixtures thereof.

According to one embodiment of the invention, the composition comprises at least one preferably modified organophilic clay, chosen from montmorillonite, bentonite, hectorite, attapulgite, sepiolite, and mixtures thereof. The clay is more advantageously a bentonite or a hectorite.

These clays are modified with a chemical compound chosen from quaternary amines, tertiary amines, amine acetates, imidazolines, amine soaps, fatty sulfates, alkylarylsulfonates and amine oxides, and mixtures thereof.

Mention may thus be made of hectorites modified with a quaternary amine, more specifically with a C₁₀ to C₂₂ fatty acid ammonium halide, such as a chloride, such as hectorite modified with distearyldimethylammonium chloride (CTFA name: Disteardimonium hectorite), for instance the product sold under the name Bentone 38V®, Bentone 38V CG or Bentone EW CE by the company Elementis, or stearalkonium hectorites, such as Bentone 27 V.

Mention may also be made of quaternium-18 bentonites, such as those sold under the names Bentone 34 by the company Elementis, Tixogel VP by the company United Catalyst and Claytone 40 by the company Southern Clay; stearalkonium bentonites, such as those sold under the names Tixogel LG by the company United Catalyst and Claytone AF and Claytone APA by the company Southern Clay; or quaternium-18/benzalkonium bentonites, such as those sold under the name Claytone HT by the company Southern Clay.

According to one preferred embodiment, the thickener is chosen from optionally modified organophilic clays, in particular organophilic hectorites in particular modified with benzyldimethylammonium stearate chloride or with distearyldimethylammonium chloride.

The composition according to the invention may also comprise, as mineral thickener, at least one preferably hydrophobic silica, chosen from fumed silicas, which are preferably hydrophobically treated, silica aerogels, and mixtures thereof.

Mention may also be made, for example, of fumed silica preferably hydrophobically treated at the surface, the size of the particles of which is more particularly less than 1 µm. Silanol groups can in particular be replaced by hydrophobic groups: a hydrophobic silica is then obtained. The hydrophobic groups can be:
- trimethylsiloxyl groups, which are obtained in particular by treating fumed silica in the presence of hexamethyldisilazane. Silicas thus treated are known as "Silica silylate" according to the CTFA (6th edition, 1995). They are sold, for example, under the references Aerosil R812® by the company Degussa, and Cab-O-Sil TS-530® by the company Cabot;
- dimethylsilyloxyl or polydimethylsiloxane groups, which are obtained in particular by treating fumed silica in the presence of polydimethylsiloxane or dimethyldichlorosilane. Silicas thus treated are known as "Silica dimethyl silylate" according to the CTFA (6th edition, 1995). They are sold, for example, under the references Aerosil R972® and Aerosil R974® by the company Degussa, and Cab-O-Sil TS-610® and Cab-O-Sil TS-720® by the company Cabot.

The hydrophobic fumed silica in particular has a particle size ranging, for example, from 5 to 200 nm.

Silica aerogels are porous materials obtained by replacing (by drying) the liquid component of a silica gel with air.

They are generally synthesized via a sol-gel process in a liquid medium and then dried, usually by extraction with a supercritical fluid, the one most commonly used being supercritical CO₂. Drying of this type makes it possible to avoid contraction of the pores and of the material. The sol-gel process and the various drying operations are described in detail in Brinker C.J., and Scherer G.W., Sol-Gel Science, New York, Academic Press, 1990.

The hydrophobic silica aerogel particles that can be used have a specific surface area per unit mass (S_{M}) ranging from 500 to 1500 m²/g, preferably from 600 to 1200 m²/g and better still from 600 to 800 m²/g, and a size expressed as the volume mean diameter (D[0.5]) ranging from 1 to 1500 µm, better still from 1 to 1000 µm, preferably from 1 to 100 µm, in particular from 1 to 30 µm, more preferably from 5 to 25 µm, better still from 5 to 20 µm and even better still from 5 to 15 µm.

According to one embodiment, the hydrophobic silica aerogel particles that are suitable have a size expressed as the volume mean diameter (D[0.5]) ranging from 1 to 30 µm, preferably from 5 to 25 µm, better still from 5 to 20 µm and even better still from 5 to 15 µm.

The specific surface area per unit mass may be determined by the nitrogen absorption method, known as the BET (Brunauer-Emmett-Teller) method, described in The Journal of the American Chemical Society, vol. 60, page 309, February 1938 and corresponding to international standard ISO 5794/1 (appendix D). The BET specific surface area corresponds to the total specific surface area of the particles under consideration.

The sizes of the silica aerogel particles can be measured by static light scattering using a commercial particle size analyser of MasterSizer 2000 type from Malvern. The data are processed on the basis of the Mie scattering theory. This theory, which is exact for isotropic particles, makes it possible to determine, in the case of non-spherical particles, an "effective" particle diameter. This theory is in particular described in the publication by Van de Hulst, H.C., Light Scattering by Small Particles, Chapters 9 and 10, Wiley, New York, 1957.

According to an advantageous embodiment, the hydrophobic silica aerogel particles that can be used according to the invention have a specific surface area per unit mass (S_{M}) ranging from 600 to 800 m²/g and a size expressed as the volume mean diameter (D[0.5]) ranging from 5 to 20 µm and even better still from 5 to 15 µm.

The silica aerogel particles that can be used may advantageously have a tapped density σ ranging from 0.02 g/cm³ to 0.10 g/cm³, preferably from 0.03 g/cm³ to 0.08 g/cm³, preferably from 0.05 g/cm³ to 0.08 g/cm³.

In the context of the present invention, this density, known as the tapped density, may be assessed according to the following protocol:
40 g of powder are poured into a graduated measuring cylinder; the measuring cylinder is then placed on the Stav 2003 device from Stampf Volumeter; the measuring cylinder is subsequently subjected to a series of 2500 tapping actions (this operation is repeated until the difference in volume between two consecutive tests is less than 2%) and then the final volume Vf of tapped powder is measured directly on the measuring cylinder. The tapped density is determined by the ratio m/Vf, in this instance 40/Vf (Vf being expressed in cm³ and m in g).

According to a preferred embodiment, the hydrophobic silica aerogel particles that can be used have a specific surface area per unit of volume S_{V} ranging from 5 to 60 m²/cm³, preferably from 10 to 50 m²/cm³ and better still from 15 to 40 m²/cm³.

The specific surface area per unit of volume is given by the relationship: S_{V} = S_{M} x σ, where σ is the tapped density, expressed in g/cm³, and S_{M} is the specific surface area per unit mass, expressed in m²/g, as defined above.

The aerogels used according to the present invention are hydrophobic silica aerogels, preferably of silyl silica (INCI name: silica silylate).

"Hydrophobic silica" is intended to mean any silica, the surface of which is treated with silylating agents, for example with halogenated silanes, such as alkylchlorosilanes, siloxanes, in particular dimethylsiloxanes, such as hexamethyldisiloxane, or silazanes, so as to functionalize the OH groups with silyl groups Si-Rn, for example trimethylsilyl groups.

As regards the preparation of hydrophobic silica aerogel particles modified at the surface by silylation, reference may be made to the document US 7 470 725.

Use will preferably be made of hydrophobic silica aerogel particles modified at the surface by trimethylsilyl groups.

As hydrophobic silica aerogels that may be used in the invention, examples that may be mentioned include the aerogel sold under the name VM-2260 (INCI name: silica silylate), by the company Dow Corning, the particles of which have a mean size of about 1000 microns and a specific surface area per unit of mass ranging from 600 to 800 m²/g.

Mention may also be made of the aerogels sold by the company Cabot under the references Aerogel TLD 201, Aerogel OGD 201, Aerogel TLD 203, Enova® Aerogel MT 1100 and Enova Aerogel MT 1200.

Use will preferably be made of the aerogel sold under the name VM-2270 (INCI name: Silica silylate) by the company Dow Corning, the particles of which have an average size ranging from 5-15 microns and a specific surface area per unit of mass ranging from 600 to 800 m²/g.

Preferably, the mineral thickeners are chosen from organophilic clays, in particular modified hectorites.

In accordance with one particular embodiment of the invention, the content of mineral thickener, expressed as active material, ranges from 0.2% to 2% by weight, preferably from 0.3% to 1.5% by weight, relative to the weight of the composition.

### C₂-C₈ MONOALCOHOL

The compositions of the invention may comprise at least one linear or branched, preferably saturated, monoalcohol comprising from 2 to 8 carbon atoms, in particular from 2 to 6 carbon atoms, and in particular from 2 to 4 carbon atoms.

As monoalcohol, mention may be made of ethanol, isopropanol, propanol or butanol, and preferably ethanol.

When the composition contains it, the content of C₂-C₈ monoalcohol is less than or equal to 6% by weight, preferably less than or equal to 4% by weight, relative to the weight of the composition.

It may be that the composition according to the invention does not comprise any abovementioned C₂-C₈ monoalcohol.

### NON-IONIC SILICONE SURFACTANT

The composition in accordance with the invention comprises moreover at least one non-ionic silicone surfactant in particular chosen from oxyalkylenated and preferably oxyethylenated polydimethylsiloxanes, alkyl or alkoxy dimethicone copolyols, and mixtures thereof. The silicone surfactants are advantageously not silicone elastomers.

Preferably, the silicone surfactant comprises polyoxyalkylene chains, more particularly chains of polyoxyethylene or of polyoxypropylene or combinations thereof, on the main chain (side or pendent polyoxyethylene or polyoxypropylene chains).

The number of ethylene oxide units can range from 0 to 100, preferably from 2 to 50 and even more particularly from 5 to 20; the number being strictly positive when the compound contains only ethylene oxide units. The number of propylene oxide units can range from 0 to 80; preferably, the compound does not contain only propylene oxide units.

Such silicone surfactants are in particular those called PEG-10 dimethicone sold by Shin-Etsu under the name KF-6017.

Also suitable are alkyl or alkoxy dimethicone copolyols bearing an alkyl or alkoxy chain that is pendent or at the end of the silicone backbone, for example containing from 6 to 22 carbon atoms.

In particular, the surfactant may be a C₈-C₂₂ alkyl dimethicone copolyol, i.e. an oxypropylenated and/or oxyethylenated polymethyl(C₈-C₂₂)alkyldimethylmethylsiloxane.

By way of example, mention may be made of cetyl dimethicone copolyol (INCI name: Cetyl PEG/PPG-10/1 Dimethicone), such as the product sold under the name Abil EM -90 by the company Evonik Goldschmidt.

Preferably, the non-ionic silicone surfactant is chosen from oxyalkylenated and preferably oxyethylenated polydimethylsiloxanes.

The content of non-ionic silicone surfactant(s) is at least 1% by weight, relative to the weight of the composition. Preferably, the content of non-ionic silicone surfactant ranges from 1% to 3% by weight, preferably from 1% to 2% by weight, relative to the weight of the composition.

### ADDITIONAL SURFACTANT

The composition according to the invention may also comprise at least one additional hydrocarbon based surfactant, preferably non-ionic. The terms "hydrocarbon based surfactant" mean that the compound does not comprise any silicon atom.

More particularly, the additional hydrocarbon based surfactant(s) are chosen from alkyl and polyalkyl esters of poly(ethylene oxide); oxyalkylenated alcohols; alkyl and polyalkyl ethers of poly(ethylene oxide), optionally polyoxyethylenated; alkyl and polyalkyl esters of sorbitan, optionally polyoxyethylenated; alkyl and polyalkyl ethers of sorbitan; alkyl and polyalkyl glycosides or polyglycosides, in particular alkyl and polyalkyl glucosides or polyglucosides, alkyl and polyalkyl esters of sucrose, optionally polyoxyethylenated alkyl and polyalkyl esters, and preferably mono-alkyl esters, of glycerol, and optionally polyoxyethylenated alkyl and polyalkyl ethers of glycerol, and mixtures thereof. More particularly, the number of oxyalkylenated units (preferably oxyethylenated and/or oxypropylenated) is ranging from 1 to 200; the alkyl chain length is ranging from 8 to 30 carbon atoms, preferably 8 to 24 carbon atoms.

Mention may be made to:
1) Alkyl and polyalkyl esters of poly(ethylene oxide) that are preferably used are those with a number of ethylene oxide (EO) units ranging from 2 to 200. Examples that may be mentioned include stearate 40 EO, stearate 50 EO, stearate 100 EO, laurate 20 EO, laurate 40 EO and distearate 150 EO.
2) Alkyl and polyalkyl ethers of poly(ethylene oxide) that are preferably used are those with a number of ethylene oxide (EO) units ranging from 2 to 200. Examples that may be mentioned include cetyl ether 23 EO, oleyl ether 50 EO and phytosterol 30 EO.
3) As oxyalkylenated alcohols, which are in particular oxyethylenated and/or oxypropylenated, use is preferably made of fatty alcohols, and which may comprise from 1 to 150 oxyethylene and/or oxypropylene units, in particular fatty alcohols of C₈-C₂₄ and preferably of C₁₂-C₁₈, containing from 20 to 100 oxyethylene units; such as stearyl alcohol ethoxylated with 10 oxyethylene units (CTFA name Stearate-10), stearyl alcohol ethoxylated with 20 oxyethylene units (CTFA name Steareth-20), for instance Brij 78 sold by the company Uniqema, stearyl alcohol ethoxylated with 100 oxyethylene units (CTFA name Steareth-100), cetearyl alcohol ethoxylated with 30 oxyethylene units (CTFA name Ceteareth-30), and the mixture of C₁₂-C₁₅ fatty alcohols comprising 7 oxyethylene units (CTFA name C₁₂-C₁₅ Pareth-7), for instance the product sold under the name Neodol 25-7® by Shell Chemicals; behenyl alcohol ethoxylated with 100 oxyethylene units (CTFA name Beheneth-100) or in particular oxyalkylenated (oxyethylenated and/or oxypropylenated) alcohols containing from 1 to 15 oxyethylene and/or oxypropylene units, in particular ethoxylated C₈-C₂₄ and preferably C₁₂-C₁₈ fatty alcohols, such as stearyl alcohol ethoxylated with 2 oxyethylene units (CTFA name Steareth-2), for instance Brij 72 sold by the company Uniqema; stearyl alcohol ethoxylated with 20 oxyethylene units (CTFA name Steareth-20), for instance Brij 78 sold by the company Uniqema.
4) Optionally polyoxyethylenated alkyl and polyalkyl esters of sorbitan that are preferably used include those with a number of ethylene oxide (EO) units ranging from 0 to 100. Examples that may be mentioned include sorbitan laurate 4 or 20 EO, in particular polysorbate 20 (or polyoxyethylene (20) sorbitan monolaurate) such as the product Tween 20 sold by the company Uniqema, sorbitan palmitate 20 EO, sorbitan stearate 20 EO, sorbitan oleate 20 EO, or the Cremophor products (RH 40, RH 60, etc.) from BASF.
5) Optionally polyoxyethylenated alkyl and polyalkyl ethers of sorbitan that are preferably used include those with a number of ethylene oxide (EO) units ranging from 0 to 100.
6) Alkyl and polyalkyl glucosides or polyglucosides that are preferably used are those containing an alkyl group comprising from 6 to 30 carbon atoms and preferably from 6 to 18 or even from 8 to 16 carbon atoms, and containing a glucoside group preferably comprising from 1 to 5 and in particular 1, 2 or 3 glucoside units. The alkylpolyglucosides may be chosen, for example, from decylglucoside (alkyl-C₉/C₁₁-polyglucoside (1.4)), for instance the product sold under the name Mydol 10® by the company Kao Chemicals or the product sold under the name Plantacare 2000 UP® by the company Henkel and the product sold under the name Oramix NS 10® by the company SEPPIC; caprylyl/capryl glucoside, for instance the product sold under the name Plantacare KE 3711® by the company Cognis or Oramix CG 110® by the company SEPPIC; laurylglucoside, for instance the product sold under the name Plantacare 1200 UP® by the company Henkel or Plantaren 1200 N® by the company Henkel; cocoglucoside, for instance the product sold under the name Plantacare 818 UP® by the company Henkel; caprylylglucoside, for instance the product sold under the name Plantacare 810 UP® by the company Cognis; and mixtures thereof.
7) Examples of alkyl and polyalkyl esters of sucrose that may be mentioned are Crodesta F150, sucrose monolaurate sold under the name Crodesta SL 40, and the products sold by Ryoto Sugar Ester, for instance sucrose palmitate sold under the reference Ryoto Sugar Ester P1670, Ryoto Sugar Ester LWA1695 or Ryoto Sugar Ester 01570.
8) Optionally polyoxyethylenated alkyl and polyalkyl esters, and preferably mono-alkyl esters, of glycerol that are preferably used are those with a number of ethylene oxide (EO) units ranging from 0 to 100 and a number of glycerol units ranging from 1 to 30. Examples that may be mentioned include hexaglyceryl monolaurate, PEG-30 glyceryl stearate, polyglyceryl 4-isostearate and glyceryl stearate.
9) Optionally polyoxyethylenated alkyl and polyalkyl ethers of glycerol that are preferably used include those with a number of ethylene oxide (EO) units ranging from 0 to 100 and a number of glycerol units ranging from 1 to 30. Examples that may be mentioned include Nikkol Batyl Alcohol 100 and Nikkol Chimyl Alcohol 100.

If the composition comprises additional hydrocarbon based surfactant(s), then the amount thereof is such that the advantageous properties of the composition according to the invention are not, or are not substantially, adversely affected by the envisioned addition. More particularly, the amount additional hydrocarbon based surfactant(s) is such that the total amount of surfactants is of at most 3% by weight, relative to the weight of the composition.

Moreover, advantageously, the ratio of the amount of additional(s) hydrocarbon based surfactant(s) relative to the total amount of silicon surfactant(s) and additional hydrocarbon based surfactant(s) is less than or equal to 0.7, preferably less than or equal to 0.5 and more particularly less than or equal to 0.2 and even more preferably less than 0.1.

According to a preferred embodiment of the invention, the composition does not comprise any additional hydrocarbon surfactant.

### SOLID PARTICLES

As indicated previously, the composition according to the invention comprises at least coloured or non-coloured solid particles.

Preferably, the solid particles are chosen from pigments and fillers, which are preferably platelet-shaped, alone or as mixtures.

### Pigments

More particularly, mineral, organic or composite pigments, and mixtures thereof, are referred to as pigments.

The term "pigments" should be understood as meaning white or coloured particles or particles which afford a colour effect, which are insoluble in the medium of the composition, and which are intended to colour and/or opacify the composition and/or the deposit produced with the composition.

The pigments may thus be chosen from monochromatic mineral pigments, organic lakes, nacres, and pigments with an optical effect, for instance reflective pigments and goniochromatic pigments.

The mineral pigments may be chosen from metal oxide pigments, chromium oxides, iron oxides, titanium dioxide, zinc oxides, cerium oxides, zirconium oxides, manganese violet, Prussian blue, ultramarine blue, ferric blue and chromium hydrate, and mixtures thereof.

They may also be pigments having a structure that may be, for example, of sericite/brown iron oxide/titanium dioxide/silica type. Such a pigment is sold, for example, under the reference Coverleaf NS or JS by the company Chemicals and Catalysts, and has a contrast ratio in the region of 30.

Organic lakes are organic pigments formed from a dye attached to a substrate.

They may be chosen, for example, from:
- cochineal carmine;
- organic pigments of azo dyes, anthraquinone dyes, indigoid dyes, xanthene dyes, pyrene dyes, quinoline dyes, triphenylmethane dyes or fluoran dyes. Among the organic pigments that may in particular be mentioned are those known under the following names: D&C Blue No. 4, D&C Brown No. 1, D&C Green No. 5, D&C Green No. 6, D&C Orange No. 4, D&C Orange No. 5, D&C Orange No. 10, D&C Orange No. 11, D&C Red No. 6, D&C Red No. 7, D&C Red No. 17, D&C Red No. 21, D&C Red No. 22, D&C Red No. 27, D&C Red No. 28, D&C Red No. 30, D&C Red No. 31, D&C Red No. 33, D&C Red No. 34, D&C Red No. 36, D&C Violet No. 2, D&C Yellow No. 7, D&C Yellow No. 8, D&C Yellow No. 10, D&C Yellow No. 11, FD&C Blue No. 1, FD&C Green No. 3, FD&C Red No. 40, FD&C Yellow No. 5, FD&C Yellow No. 6;
- insoluble sodium, potassium, calcium, barium, aluminium, zirconium, strontium or titanium salts of acid dyes such as azo, anthraquinone, indigoid, xanthene, pyrene, quinoline, triphenylmethane or fluoran dyes, these dyes possibly comprising at least one carboxylic or sulfonic acid group.

The organic lakes may also be supported on an organic support such as rosin or aluminium benzoate, for example.

Among the organic lakes that may be mentioned in particular are those known under the following names: D&C Red No. 2 Aluminium lake, D&C Red No. 3 Aluminium lake, D&C Red No. 4 Aluminium lake, D&C Red No. 6 Aluminium lake, D&C Red No. 6 Barium lake, D&C Red No. 6 Barium/Strontium lake, D&C Red No. 6 Strontium lake, D&C Red No. 6 Potassium lake, D&C Red No. 7 Aluminium lake, D&C Red No. 7 Barium lake, D&C Red No. 7 Calcium lake, D&C Red No. 7 Calcium/Strontium lake, D&C Red No. 7 Zirconium lake, D&C Red No. 8 Sodium lake, D&C Red No. 9 Aluminium lake, D&C Red No. 9 Barium lake, D&C Red No. 9 Barium/Strontium lake, D&C Red No. 9 Zirconium lake, D&C Red No. 10 Sodium lake, D&C Red No. 19 Aluminium lake, D&C Red No. 19 Barium lake, D&C Red No. 19 Zirconium lake, D&C Red No. 21 Aluminium lake, D&C Red No. 21 Zirconium lake, D&C Red No. 22 Aluminium lake, D&C Red No. 27 Aluminium lake, D&C Red No. 27 Aluminium/Titanium/Zirconium lake, D&C Red No. 27 Barium lake, D&C Red No. 27 Calcium lake, D&C Red No. 27 Zirconium lake, D&C Red No. 28 Aluminium lake, D&C Red No. 30 lake, D&C Red No. 31 Calcium lake, D&C Red No. 33 Aluminium lake, D&C Red No. 34 Calcium lake, D&C Red No. 36 lake, D&C Red No. 40 Aluminium lake, D&C Blue No. 1 Aluminium lake, D&C Green No. 3 Aluminium lake, D&C Orange No. 4 Aluminium lake, D&C Orange No. 5 Aluminium lake, D&C Orange No. 5 Zirconium lake, D&C Orange No. 10 Aluminium lake, D&C Orange No. 17 Barium lake, D&C Yellow No. 5 Aluminium lake, D&C Yellow No. 5 Zirconium lake, D&C Yellow No. 6 Aluminium lake, D&C Yellow No. 7 Zirconium lake, D&C Yellow No. 10 Aluminium lake, FD&C Blue No. 1 Aluminium lake, FD&C Red No. 4 Aluminium lake, FD&C Red No. 40 Aluminium lake, FD&C Yellow No. 5 Aluminium lake, FD&C Yellow No. 6 Aluminium lake.

The chemical materials corresponding to each of the organic dyestuffs mentioned previously are mentioned in the publication "International Cosmetic Ingredient Dictionary and Handbook", 1997 edition, pages 371 to 386 and 524 to 528, published by The Cosmetic, Toiletries and Fragrance Association, the content of which is incorporated into the present patent application by reference.

The pigments may also have been subjected to a hydrophobic treatment.

The hydrophobic treatment agent may be chosen from silicones such as methicones, dimethicones and perfluoroalkylsilanes; fatty acids such as stearic acid; metal soaps such as aluminium dimyristate, the aluminium salt of hydrogenated tallow glutamate, perfluoroalkyl phosphates, perfluoroalkylsilanes, perfluoroalkylsilazanes, polyhexafluoropropylene oxides, polyorganosiloxanes comprising perfluoroalkyl perfluoropolyether groups and amino acids; N-acylamino acids or salts thereof; lecithin, isopropyl triisostearyl titanate, and mixtures thereof.

The N-acylamino acids may comprise an acyl group containing from 8 to 22 carbon atoms, for instance a 2-ethylhexanoyl, caproyl, lauroyl, myristoyl, palmitoyl, stearoyl or cocoyl group. The salts of these compounds may be the aluminium, magnesium, calcium, zirconium, zinc, sodium or potassium salts. The amino acid may be, for example, lysine, glutamic acid or alanine.

The term "alkyl" mentioned in the compounds cited above in particular denotes an alkyl group containing from 1 to 30 carbon atoms and preferably containing from 5 to 16 carbon atoms.

Hydrophobic treated pigments are described in particular in patent application EP-A-1 086 683.

The dyestuff may also comprise a pigment having a structure which may be, for example, of the type such as silica microspheres containing iron oxide. An example of a pigment having this structure is the product sold by the company Miyoshi under the reference PC Ball PC-LL-100 P, this pigment being constituted of silica microspheres containing yellow iron oxide.

For the purposes of the present patent invention, the nacres are more particularly coloured particles of any form, which may or may not be iridescent, in particular produced by certain molluscs in their shell, or alternatively synthesized, and which have a colour effect via optical interference.

Examples of nacres that may be mentioned include nacreous pigments such as titanium mica coated with an iron oxide, mica coated with bismuth oxychloride, titanium mica coated with chromium oxide, titanium mica coated with an organic dye in particular of the abovementioned type, and also nacreous pigments based on bismuth oxychloride. They may also be mica particles, on the surface of which are superposed at least two successive layers of metal oxides and/or of organic dyestuffs.

The nacres may more particularly have a yellow, pink, red, bronze, orange, brown, gold and/or coppery colour or tint.

As illustrations of nacres that may be introduced as interference pigments into the first composition, mention may be made of the gold-coloured nacres sold in particular by the company Engelhard under the name Brilliant gold 212G (Timica), Gold 222C (Cloisonne), Sparkle gold (Timica), Gold 4504 (Chromalite) and Monarch gold 233X (Cloisonne); the bronze nacres sold in particular by the company Merck under the name Bronze fine (17384) (Colorona) and Bronze (17353) (Colorona) and by the company Engelhard under the name Super bronze (Cloisonne); the orange nacres sold in particular by the company Engelhard under the name Orange 363C (Cloisonne) and Orange MCR 101 (Cosmica) and by the company Merck under the name Passion orange (Colorona) and Matte orange (17449) (Microna); the brown nacres sold in particular by the company Engelhard under the name Nu-antique copper 340XB (Cloisonne) and Brown CL4509 (Chromalite); the nacres with a copper tint sold in particular by the company Engelhard under the name Copper 340A (Timica); the nacres with a red tint sold in particular by the company Merck under the name Sienna fine (17386) (Colorona); the nacres with a yellow tint sold in particular by the company Engelhard under the name Yellow (4502) (Chromalite); the red nacres with a gold tint sold in particular by the company Engelhard under the name Sunstone G012 (Gemtone); the pink nacres sold in particular by the company Engelhard under the name Tan opale G005 (Gemtone); the black nacres with a gold tint sold in particular by the company Engelhard under the name Nu antique bronze 240 AB (Timica), the blue nacres sold in particular by the company Merck under the name Matte blue (17433) (Microna), the white nacres with a silvery tint sold in particular by the company Merck under the name Xirona Silver, and the golden-green pink-orange nacres sold in particular by the company Merck under the name Indian summer (Xirona), and mixtures thereof.

The composition may also contain, as coloured particles, at least one material with a specific optical effect.

This effect is different from a simple conventional hue effect, i.e. a unified and stabilized effect as produced by standard coloured particles, for instance monochromatic pigments. For the purposes of the invention, the term "stabilized" is intended to mean lacking the effect of variability of the colour with the angle of observation or in response to a temperature change.

For example, this material may be chosen from particles with a metallic glint, goniochromatic colouring agents, diffractive pigments, thermochromic agents, optical brighteners, and also fibres, in particular interference fibres. Needless to say, these various materials may be combined so as to afford the simultaneous manifestation of two effects, or even of a novel effect.

Advantageously, the content of coloured particles, in particular of pigments, of nacres alone or as mixtures, ranges from 0.001% to 10% by weight and preferably from 0.01% to 8% by weight relative to the weight of the composition.

### Fillers

The fillers are more particularly organic, mineral or mixed, and may be present alone or as a mixture.

The term "fillers" should be understood as meaning colourless or white solid particles of any form, which are in an insoluble and dispersed form in the medium of the composition, and which are not intended to colour the composition, but at the very most to opacify it.

The fillers may be, for example, platelet-shaped, spherical, oblong or fibrous, or of any other form intermediate between these forms, irrespective of the crystallographic form (for example sheet, cubic, hexagonal, orthorhombic, etc.). Preferably, the fillers used are platelet -shaped.

It should be noted that the fillers are different from the mineral thickener previously mentioned.

The fillers according to the invention may or may not be surface-coated, and in particular they may be surface-treated with silicones, amino acids, fluoro derivatives or any other substance that promotes the dispersion and compatibility of the filler in the composition.

Examples of mineral fillers that may be mentioned include talc, mica, perlite, kaolin, hollow silica microspheres, precipitated calcium carbonate, magnesium carbonate, magnesium hydrogen carbonate, hydroxyapatite, boron nitride, glass or ceramic microcapsules, or composites of silica and of titanium dioxide, for instance the TSG series sold by Nippon Sheet Glass.

Examples of organic fillers that may be mentioned include polyamide powders (Nylon® Orgasol from Atochem), polyethylene powders, polymethyl methacrylate powders, polytetrafluoroethylene (Teflon) powders, acrylic acid copolymer powders (Polytrap from the company Dow Corning), lauroyllysine, hollow polymer microspheres such as those of polyvinylidene chloride/acrylonitrile, for instance Expancel (Nobel Industrie), hexamethylene diisocyanate/trimethylol hexyl lactone copolymer powder (Plastic Powder from Toshiki), silicone resin microbeads (for example Tospearls from Toshiba), synthetic or natural micronized waxes, metal soaps derived from organic carboxylic acids containing from 8 to 22 carbon atoms and preferably from 12 to 18 carbon atoms, for example zinc stearate, magnesium stearate, lithium stearate, zinc laurate or magnesium myristate, Polypore® L 200 (Chemdal Corporation), polyurethane powders, in particular powders of crosslinked polyurethane comprising a copolymer, said copolymer comprising trimethylol hexyl lactone. It may in particular be a hexamethylene diisocyanate/trimethylol hexyllactone polymer. Such particles are in particular commercially available, for example, under the name Plastic Powder D-400® or Plastic Powder D-800® from the company Toshiki, and mixtures thereof.

As organic filler, mention may also be made of powders of crosslinked organopolysiloxane coated with silicone resin, in particular with silsesquioxane resin, as described, for example, in patent US 5 538 793.

Such elastomer powders are sold under the names KSP-100®, KSP-101®, KSP-102®, KSP-103®, KSP-104® and KSP-105® by the company Shin-Etsu; mention may also be made of crosslinked organopolysiloxane elastomer powders coated with silicone resin, such as powders of hybrid silicone functionalized with fluoroalkyl groups, sold in particular under the name KSP-200 by the company Shin-Etsu; or hybrid silicone powders functionalized with phenyl groups, sold in particular under the name KSP-300 by the company Shin-Etsu.

Preferably, use is made of mineral fillers, which are advantageously platelet -shaped, and even more preferentially mica.

According to one particular embodiment of the invention, the content of fillers ranges from 0.001% to 5% by weight, preferably from 0.05% to 3% by weight, relative to the weight of the composition.

### WATER

The composition according to the invention can optionally comprise water.

If it contains water, the water content does not exceed 1% by weight, more particularly does not exceed 0.5% by weight, and preferably does not exceed 0.4% by weight, relative to the weight of the composition.

### VOLATILE OILS

The composition according to the invention may also comprise at least one volatile oil.

Preferably, said volatile oil is chosen from volatile hydrocarbon-based oils, which are preferably non-polar, and volatile silicone oils.

For example, the volatile silicone oil(s) can be chosen in particular from silicone oils with a flash point ranging from 40°C to 102°C, preferably greater than 55°C and less than or equal to 95°C, and even more particularly from 65°C to 95°C.

As volatile silicone oils that may be used in the invention, mention may be made of linear or cyclic silicones containing in particular from 2 to 10 silicon atoms and in particular from 2 to 7 silicon atoms, these silicones optionally comprising alkyl or alkoxy groups containing from 1 to 10 carbon atoms. As volatile silicone oils, mention may be made of dimethicones with viscosities of 5 and 6 cSt, octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

The volatile hydrocarbon-based oil(s), which are preferably non-polar, are chosen from oils of which the flash point ranges from 40°C to 102°C, preferably from 40°C to 55°C, and even more particularly from 40°C to 50°C.

Among the volatile hydrocarbon-based oils that can be used, mention may in particular be made of hydrocarbons containing from 8 to 16 carbon atoms, and mixtures thereof, and in particular:
- branched C₈-C₁₆ alkanes such as C₈-C₁₆ isoalkanes (also known as isoparaffins), isododecane, isodecane and isohexadecane, and, for example, the oils sold under the trade name Isopar or Permethyl,
- linear alkanes, for instance n-dodecane (C12) and n-tetradecane (C14) sold by Sasol under the respective references Parafol 12-97 and Parafol 14-97, and also mixtures thereof, the undecane-tridecane mixture (Cetiol UT), the mixtures of n-undecane (C11) and of n-tridecane (C13) obtained in Examples 1 and 2 of patent application WO 2008/155 059 from the company Cognis, and mixtures thereof.

In accordance with one advantageous embodiment of the invention, the composition comprises at least one volatile hydrocarbon-based oil, which is more particularly non-polar, at least one volatile silicone oil, and preferably a mixture of these two types of volatile oils.

More particularly, the content of volatile hydrocarbon-based oil(s), volatile silicone oil(s) or mixtures thereof is less than or equal to 10% by weight, more particularly less than or equal to 8% by weight, advantageously between 1% and 8% by weight, relative to the weight of the composition.

If the composition comprises a mixture of non-volatile oil(s) and volatile oil(s), then said mixture is single-phase. More particularly, the mixture is said to be single-phase when no phase separation is observed by eye or under a phase-contrast microscope, at ambient temperature (25°C) after homogenization at temperature and mixing on a Rayneri mixer (550 rpm, 10 minutes) and storage while left to stand in a closed receptacle at ambient temperature (and atmospheric pressure) for 24 hours.

### SILICONE POLYMER

The composition according the invention may optionally comprise at least one silicone polymer chosen from:
(i) silicone resins of MQ type;
(ii) silsesquioxanes resins;
(iii) vinyl polymers grafted with a carbosiloxane dendrimer;
(iv) mixtures thereof.

The term "polymer" is intended to mean herein a compound containing one or more repeating unit(s) and preferably at least 2 repeating units.

### Silicone resins of MQ type

More generally, the term "resin" is intended to mean a compound whose structure is three-dimensional.

"Silicone resins" are also referred to as "siloxane resins". Thus, for the purposes of the present invention, a polydimethylsiloxane is not a silicone resin.

The nomenclature of silicone resins is known under the name "MDTQ", the resin being described as a function of the various siloxane monomer units that it comprises, each of the letters "MDTQ" characterizing a type of unit.

The letter M represents the monofunctional unit of formula R¹R²R³SiO_{1/2}, the silicon atom being connected to only one oxygen atom in the polymer comprising this unit.

The letter "D" signifies a difunctional unit R¹R²SiO_{2/2} in which the silicon atom is bonded to two oxygen atoms.

The letter "T" represents a trifunctional unit of formula R¹SiO_{3/2}.

Finally, the letter "Q" signifies a tetrafunctional unit SiO_{4/2} in which the silicon atom is bonded to four oxygen atoms, which are themselves bonded to the rest of the polymer.

In the units M, D and T defined previously, R¹, R² and R³ represent a hydrocarbon-based radical (in particular alkyl) containing from 1 to 10 carbon atoms, a phenyl group, a phenylalkyl group or even a hydroxyl group.

Such resins are described for example in Encyclopedia of Polymer Science and Enginnering, vol. 15, John Wiley and Sons, New York, (1989), p. 265-270, and US 2 676 182, US 3 627 851, US 3 772 247, US 5 248 739 or else US 5 082 706, US 5 319 040, US 5 302 685 and US 4 935 484.

As examples of silicone resins of MQ type that can be used according to the invention, mention may be made of the alkyl siloxysilicates of formula [(R₁)₃SiO_{1/2}]x(SiO_{4/2})_{y} (MQ units) in which x and y are integers ranging from 50 to 80, and such that the group R₁ represents a radical as defined previously, and is preferably an alkyl group containing from 1 to 8 carbon atoms or a hydroxyl group, preferably a methyl group.

As examples of solid silicone resins of MQ type of trimethyl siloxysilicate type, mention may be made of those sold under the reference SR1000 by the company General Electric, under the reference TMS 803 by the company Wacker, or under the name KF-7312J by the company Shin-Etsu or DC749 or DC593 by the company Dow Corning.

As silicone resins comprising MQ siloxysilicate units, mention may also be made of phenylalkylsiloxysilicate resins, such as phenylpropyldimethylsiloxysilicate (Silshine 151 sold by the company General Electric). The preparation of such resins is described in particular in patent US 5 817 302.

### Silsesquioxane resins

Among the silsesquioxane resins that can be used in the compositions in accordance with the invention, mention may be made of the alkyl silsesquioxane resins which are homopolymers and/or copolymers of silsesquioxane containing an average siloxane unit of formula R₁ₙSiO_{(4-n)/2}, in which each R₁ independently denotes a hydrogen atom or a C₁-C₁₀ alkyl group, where more than 80 mol% of the R₁ radicals represent a C₃-C₁₀ alkyl group, n is a number from 1.0 to 1.4, and more particularly, use will be made of a silsesquioxane copolymer in which more than 60 mol% comprises units R₁SiO_{3/2} in which R₁ has the definition previously indicated.

Preferably, the silsesquioxane resin is chosen such that R₁ is a C₁-C₁₀ alkyl group, preferably a C₁-C₄ alkyl group, and more particularly a propyl group. Use will more particularly made of a polypropylsilsesquioxane or t-propyl silsesquioxane resin (INCI name: Polypropylsilsesquisiloxane (and) Isododecane) such as the product sold under the trade name Dow Corning® 670 Fluid by the company Dow Corning.

### Vinyl polymers grafted with a carbosiloxane dendrimer

Among the silicone polymers that are suitable for the composition according to the invention, mention may be made of vinyl polymers comprising at least one carbosiloxane dendrimer-based unit.

The vinyl polymer has a backbone and at least one side chain, which comprises a carbosiloxane dendrimer-based unit having a carbosiloxane dendrimer structure.

In the context of the present invention, the term "carbosiloxane dendrimer structure" represents a molecular structure containing branched groups of high molecular masses, said structure having high regularity in the radial direction starting from the bond to the backbone. Such carbosiloxane dendrimer structures are described in the form of a highly branched siloxane-silylalkylene copolymer in laid-open Japanese patent application Kokai 9-171 154.

A vinyl polymer according to the invention may contain carbosiloxane dendrimer-based units that may be represented by the following general formula (I): 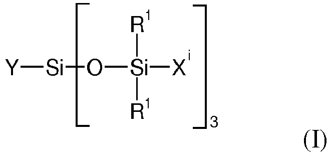 in which:
- R¹ represents an aryl group containing from 5 to 10 carbon atoms or an alkyl group containing from 1 to 10 carbon atoms;
- Xⁱ represents a silylalkyl group which, when i = 1, is represented by formula (II): 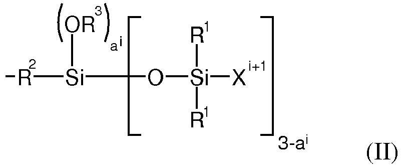 in which:
   - R¹ is as defined above in formula (I),
   - R² represents an alkylene radical containing from 2 to 10 carbon atoms,
   - R³ represents an alkyl group containing from 1 to 10 carbon atoms,
   - Xⁱ⁺ⁱ is chosen from: a hydrogen atom, an alkyl group containing from 1 to 10 carbon atoms, an aryl group containing from 5 to 10 carbon atoms and a silylalkyl group defined above of formula (II) with i = i + 1,
   - i is an integer from 1 to 10 which represents the generation of said silylalkyl group, and
   - aⁱ is an integer from 0 to 3;
- Y represents a radical-polymerizable organic group chosen from:
   - organic groups containing a methacrylic group or an acrylic group, said organic groups being represented by the formulae: 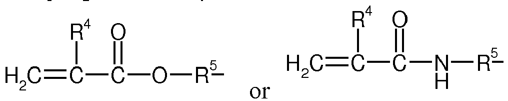 in which:
      * R⁴ represents a hydrogen atom or an alkyl group containing from 1 to 10 carbon atoms; and
      * R⁵ represents an alkylene group containing from 1 to 10 carbon atoms, such as a methylene group, an ethylene group, a propylene group or a butylene group, methylene and propylene groups being preferred; and
   - organic groups containing a styryl group of formula: 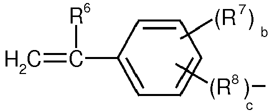 in which:
      * R⁶ represents a hydrogen atom or an alkyl group containing from 1 to 10 carbon atoms, such as a methyl group, an ethyl group, a propyl group or a butyl group, the methyl group being preferred;
      * R⁷ represents an alkyl group containing from 1 to 10 carbon atoms;
      * R⁸ represents an alkylene group containing from 1 to 10 carbon atoms, such as a methylene group, an ethylene group, a propylene group or a butylene group, the ethylene group being preferred;
      * b is an integer from 0 to 4; and
      * c is 0 or 1, such that, if c is 0, -(R⁸)c- represents a bond.

According to one embodiment, R¹ may represent an aryl group containing from 5 to 10 carbon atoms or an alkyl group containing from 1 to 10 carbon atoms. The alkyl group may preferably be represented by a methyl group, an ethyl group, a propyl group, a butyl group, a pentyl group, an isopropyl group, an isobutyl group, a cyclopentyl group or a cyclohexyl group. The aryl group may preferably be represented by a phenyl group and a naphthyl group. The methyl and phenyl groups are more particularly preferred, and the methyl group is preferred among all.

According to one embodiment, R² represents an alkylene group containing from 2 to 10 carbon atoms, in particular a linear alkylene group, such as an ethylene, propylene, butylene or hexylene group; or a branched alkylene group, such as a methylmethylene, methylethylene, 1-methylpentylene or 1,4-dimethylbutylene group.

The ethylene, methylethylene, hexylene, 1-methylpentylene and 1,4-dimethylbutylene groups are preferred among all.

According to one embodiment, R³ is chosen from methyl, ethyl, propyl, butyl and isopropyl groups.

In formula (II), i indicates the number of generations and thus corresponds to the number of repeats of the silylalkyl group.

For example, when the generation number is equal to one, the carbosiloxane dendrimer may be represented by the general formula shown below, in which Y, R¹, R² and R³ are as defined above, R¹² represents a hydrogen atom or is identical to R¹; a¹ is identical to aⁱ. Preferably, the total average number of groups OR³ in a molecule is within the range from 0 to 7.

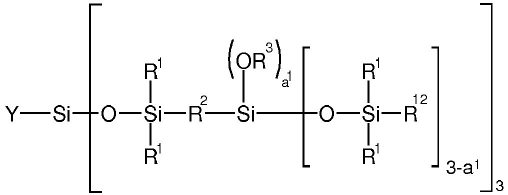

When the generation number is equal to 2, the carbosiloxane dendrimer may be represented by the general formula below, in which Y, R¹, R², R³ and R¹² are the same as defined above; a¹ and a² represent the ai of the indicated generation. Preferably, the total average number of groups OR³ in a molecule is within the range from 0 to 25.

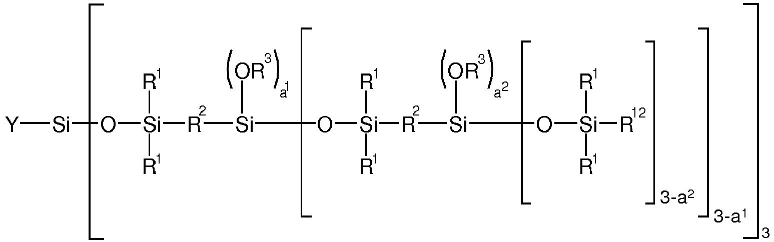

When the generation number is equal to 3, the carbosiloxane dendrimer is represented by the general formula below, in which Y, R¹, R², R³ and R¹² are the same as defined above; a¹, a² and a³ represent the ai of the indicated generation. Preferably, the total average number of groups OR³ in a molecule is within the range from 0 to 79.

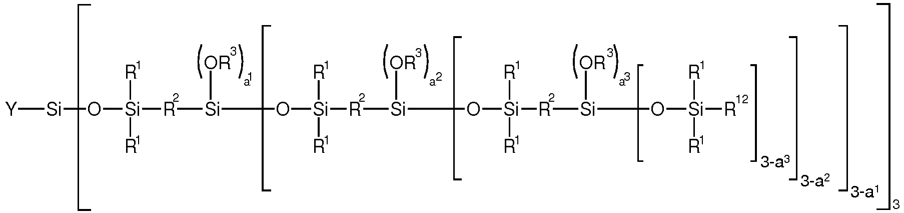

A vinyl polymer having at least one unit derived from carbosiloxane dendrimer has a molecular side chain containing a carbosiloxane dendrimer structure and can result from the polymerization of:
(A) from 0 to 99.9 parts by weight of a vinyl monomer; and
(B) from 100 to 0.1 parts by weight of a carbosiloxane dendrimer containing a radical-polymerizable organic group, represented by general formula (I) as defined above.

The monomer of vinyl type that is component (A) in the vinyl polymer containing at least one carbosiloxane dendrimer-based unit is a monomer of vinyl type that contains a radical-polymerizable vinyl group.

There is no particular limitation as regards such a monomer.

The following are examples of this monomer of vinyl type: methyl methacrylate, ethyl methacrylate, n-propyl methacrylate, isopropyl methacrylate or a methacrylate of lower alkyl analogue; glycidyl methacrylate; butyl methacrylate, butyl acrylate, n-butyl methacrylate, isobutyl methacrylate, tert-butyl acrylate, tert-butyl methacrylate, n-hexyl methacrylate, cyclohexyl methacrylate, 2-ethylhexyl acrylate, 2-ethylhexyl methacrylate, octyl methacrylate, lauryl methacrylate, stearyl acrylate, stearyl methacrylate or a higher methacrylate analogue; vinyl acetate, vinyl propionate or a vinyl ester of a lower fatty acid analogue; vinyl caproate, vinyl 2-ethylhexoate, vinyl laurate, vinyl stearate or a higher fatty acid ester analogue; styrene, vinyltoluene, benzyl methacrylate, phenoxyethyl methacrylate, vinylpyrrolidone or similar vinylaromatic monomers; methacrylamide, N-methylolmethacrylamide, N-methoxymethylmethacrylamide, isobutoxymethoxymethacrylamide, N,N-dimethylmethacrylamide or similar monomers of vinyl type containing amide groups; hydroxyethyl methacrylate, hydroxypropyl alcohol methacrylate or similar monomers of vinyl type containing hydroxyl groups; acrylic acid, methacrylic acid, itaconic acid, crotonic acid, fumaric acid, maleic acid or similar monomers of vinyl type containing a carboxylic acid group; tetrahydrofurfuryl methacrylate, butoxyethyl methacrylate, ethoxydiethylene glycol methacrylate, polyethylene glycol methacrylate, polypropylene glycol monomethacrylate, hydroxybutyl vinyl ether, cetyl vinyl ether, 2-ethylhexyl vinyl ether or a similar monomer of vinyl type with ether bonds; methacryloxypropyltrimethoxysilane, polydimethylsiloxane containing a methacrylic group on one of its molecular ends, polydimethylsiloxane containing a styryl group on one of its molecular ends, or a similar silicone compound containing unsaturated groups; butadiene; vinyl chloride; vinylidene chloride; methacrylonitrile; dibutyl fumarate; anhydrous maleic acid; anhydrous succinic acid; methacryl glycidyl ether; an organic salt of an amine, an ammonium salt, and an alkali metal salt of methacrylic acid, of itaconic acid, of crotonic acid, of maleic acid or of fumaric acid; a radical-polymerizable unsaturated monomer containing a sulfonic acid group such as a styrenesulfonic acid group; a quaternary ammonium salt derived from methacrylic acid, such as 2-hydroxy-3-methacryloxypropyltrimethylammonium chloride; and a methacrylic acid ester of an alcohol containing a tertiary amine group, such as a methacrylic acid ester of diethylamine.

Multifunctional monomers of vinyl type can also be used.

The following represent examples of such compounds: trimethylolpropane trimethacrylate, pentaerythrityl trimethacrylate, ethylene glycol dimethacrylate, tetraethylene glycol dimethacrylate, polyethylene glycol dimethacrylate, 1,4-butanediol dimethacrylate, 1,6-hexanediol dimethacrylate, neopentyl glycol dimethacrylate, trimethylolpropanetrioxyethyl methacrylate, tris(2-hydroxyethyl)isocyanurate dimethacrylate, tris(2-hydroxyethyl)isocyanurate trimethacrylate, polydimethylsiloxane capped with styryl groups containing divinylbenzene groups on both ends, or similar silicone compounds containing unsaturated groups.

A carbosiloxane dendrimer, which is the component (B), may be represented by formula (I) as defined above.

The following represent the preferred examples of group Y of formula (I): an acryloxymethyl group, a 3-acryloxypropyl group, a methacryloxymethyl group, a 3-methacryloxypropyl group, a 4-vinylphenyl group, a 3-vinylphenyl group, a 4-(2-propenyl)phenyl group, a 3-(2-propenyl)phenyl group, a 2-(4-vinylphenyl)ethyl group, a 2-(3-vinylphenyl)ethyl group, a vinyl group, an allyl group, a methallyl group and a 5-hexenyl group.

A carbosiloxane dendrimer according to the present invention may be represented by the formulae having the average structures below:

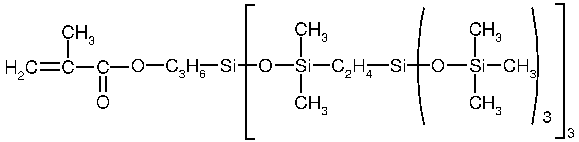

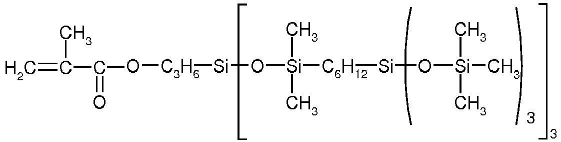

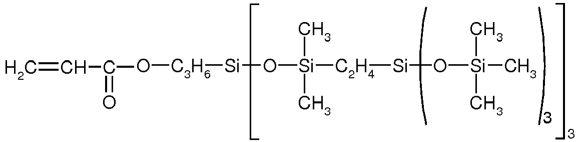

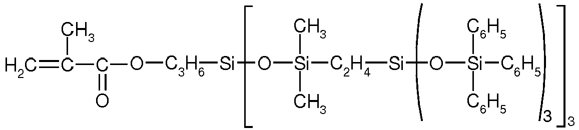

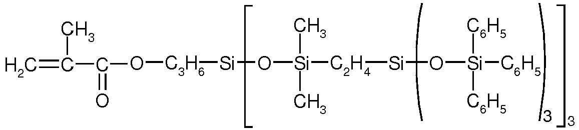

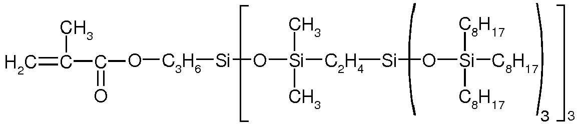

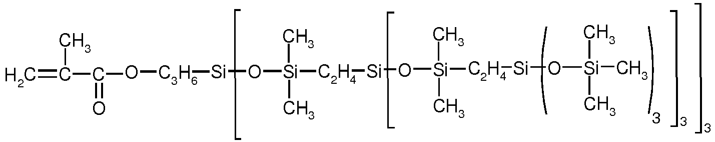

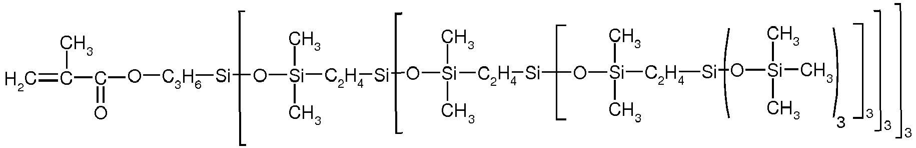

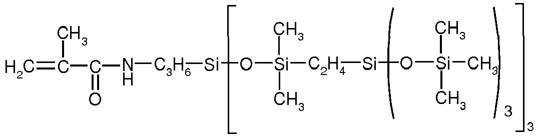

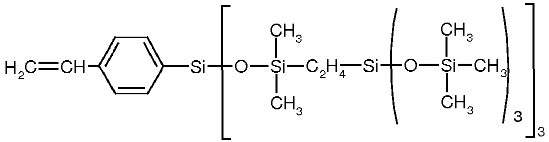

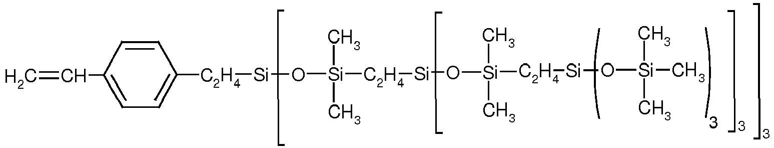

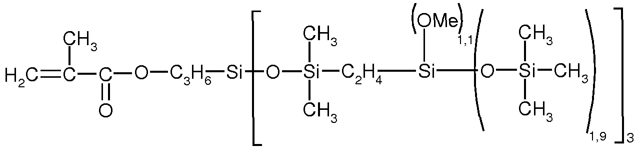

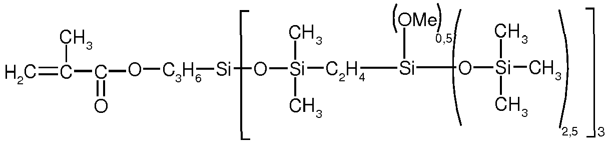

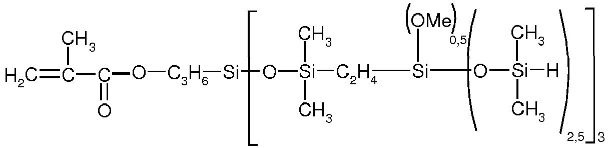

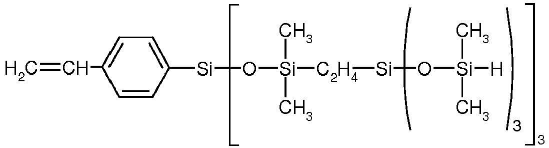

Thus, according to one embodiment, the carbosiloxane dendrimer of the composition according to the present invention is represented by the following formula: 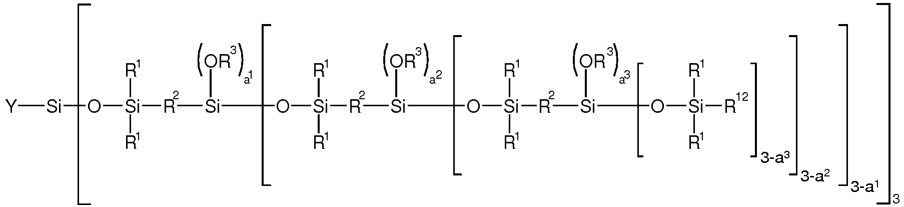 in which:
- Y, R¹, R² and R³ are as defined in formulae (I) and (II) above;
- a¹, a² and a³ correspond to the definition of ai according to formula (II); and
- R¹² is H, an aryl group containing from 5 to 10 carbon atoms or an alkyl group containing from 1 to 10 carbon atoms.

According to one embodiment, the carbosiloxane dendrimer of the composition according to the present invention is represented by one of the following formulae:

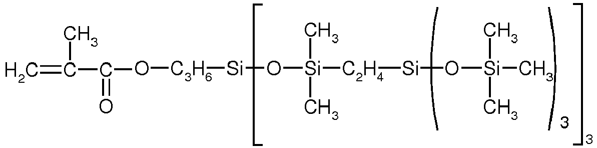

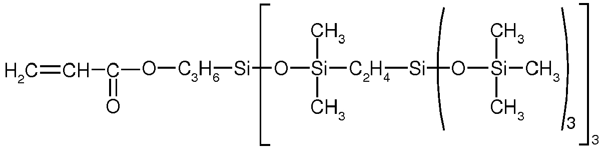

The vinyl polymer comprising the carbosiloxane dendrimer that can be used may be manufactured according to the process for manufacturing a branched silalkylene siloxane described in Japanese patent application Hei 9-171 154.

The vinyl polymer may be a dispersion of a polymer of vinyl type bearing a carbosiloxane dendrimer structure in its side molecular chain, in a liquid such as a silicone oil, an organic oil, an alcohol or water.

The silicone oil may be a dimethylpolysiloxane having the two molecular ends capped with trimethylsiloxy groups, a copolymer of methylphenylsiloxane and of dimethylsiloxane having the two molecular ends capped with trimethylsiloxy groups, a copolymer of methyl-3,3,3-trifluoropropylsiloxane and of dimethylsiloxane having the two molecular ends capped with trimethylsiloxy groups, or similar unreactive linear silicone oils, and also hexamethylcyclotrisiloxane, octamethylcyclotetrasiloxane, decamethylcyclopentasiloxane, dodecamethylcyclohexasiloxane or a similar cyclic compound. In addition to the unreactive silicone oils, modified polysiloxanes containing functional groups such as silanol groups, amino groups and polyether groups on the ends or within the molecular side chains may be used.

The organic oils may be isododecane, liquid paraffin, isoparaffin, hexyl laurate, isopropyl myristate, myristyl myristate, cetyl myristate, 2-octyldodecyl myristate; isopropyl palmitate, 2-ethylhexyl palmitate, butyl stearate, decyl oleate, 2-octyldodecyl oleate, myristyl lactate, cetyl lactate, lanolin acetate, stearyl alcohol, cetostearyl alcohol, oleyl alcohol, avocado oil, almond oil, olive oil, cocoa oil, jojoba oil, gum oil, sunflower oil, soybean oil, camelia oil, squalane, castor oil, cottonseed oil, coconut oil, egg yolk oil, polypropylene glycol monooleate, neopentyl glycol 2-ethylhexanoate or a similar glycol ester oil; triglyceryl isostearate, the triglyceride of a fatty acid of coconut oil, or a similar oil of a polyhydric alcohol ester; polyoxyethylene lauryl ether, polyoxypropylene cetyl ether or a similar polyoxyalkylene ether.

The alcohol may be any type that is suitable for use in combination with a cosmetic product starting material. For example, it may be methanol, ethanol, butanol, isopropanol or similar lower alcohols.

The solutions and dispersions may be readily prepared by mixing a vinyl polymer bearing at least one carbosiloxane dendrimer-based unit with a silicone oil, an organic oil, an alcohol or water. The liquids may be present in the polymerization step. In this case, the unreacted residual vinyl monomer should be completely removed by heat treatment of the solution or dispersion under atmospheric pressure or reduced pressure.

In the case of a dispersion, the dispersity of the polymer of vinyl type may be improved by adding a surfactant.

Such an agent may be hexylbenzenesulfonic acid, octylbenzenesulfonic acid, decylbenzenesulfonic acid, dodecylbenzenesulfonic acid, cetylbenzenesulfonic acid, myristylbenzenesulfonic acid or anionic surfactants of the sodium salts of these acids; octyltrimethylammonium hydroxide, dodecyltrimethylammonium hydroxide, hexadecyltrimethylammonium hydroxide, octyldimethylbenzylammonium hydroxide, decyldimethylbenzylammonium hydroxide, dioctadecyldimethylammonium hydroxide, beef tallow-trimethylammonium hydroxide, coconut oil-trimethylammonium hydroxide, or a similar cationic surfactant; a polyoxyalkylene alkyl ether, a polyoxyalkylenealkylphenol, a polyoxyalkylene alkyl ester, the sorbitol ester of polyoxyalkylene, polyethylene glycol, polypropylene glycol, an ethylene oxide additive of diethylene glycol trimethylnonanol, and non-ionic surfactants of polyester type, and also mixtures.

In the dispersion, a mean particle diameter of the polymer of vinyl type may be within a range of between 0.001 and 100 microns and preferably between 0.01 and 50 microns.

A vinyl polymer contained in the dispersion or the solution may have a concentration within a range of between 0.1% and 95% by weight and preferably between 5% and 85% by weight. However, to facilitate the handling and the preparation of the mixture, the range should preferably be between 10% and 75% by weight.

A vinyl polymer that is suitable for use in the invention may also be one of the polymers described in the examples of patent application EP 0 963 751.

According to a preferred embodiment, a vinyl polymer grafted with a carbosiloxane dendrimer can result from the polymerization of:
from 0 to 99.9 parts by weight of one or more acrylate or methacrylate monomers; and
from 100 to 0.1 part by weight of an acrylate or methacrylate monomer of a tris[tri(trimethylsiloxy)silylethyldimethylsiloxy]silylpropyl carbosiloxane dendrimer.

The monomers (A1) and (B1) correspond respectively to specific monomers (A) and (B).

According to one embodiment, a vinyl polymer bearing at least one carbosiloxane dendrimer-based unit may comprise a tris[tri(trimethylsiloxy)silylethyldimethylsiloxy] silylpropyl carbosiloxane dendrimer-based unit corresponding to one of the formulae: 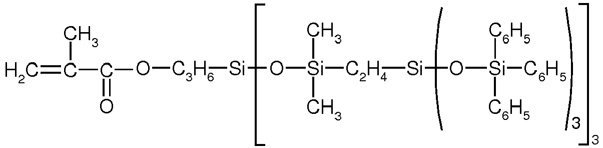 or

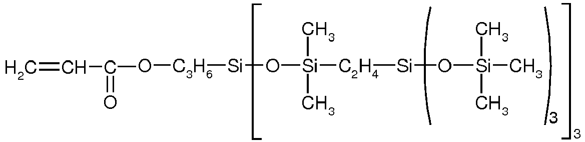

According to one preferred mode, a vinyl polymer bearing at least one carbosiloxane dendrimer-based unit used in the invention comprises at least one butyl acrylate monomer.

According to one embodiment, a vinyl polymer can additionally comprise at least one fluorinated organic group.

Structures in which the polymerized vinyl units constitute the backbone and carbosiloxane dendritic structures and also fluoro organic groups are attached to side chains are particularly preferred.

The fluoro organic groups may be obtained by replacing with fluorine atoms all or some of the hydrogen atoms of methyl, ethyl, propyl, isopropyl, butyl, isobutyl, tert-butyl, pentyl, neopentyl, hexyl, cyclohexyl, heptyl, octyl, nonyl, decyl, undecyl, dodecyl, tridecyl, tetradecyl, hexadecyl and octadecyl groups and other alkyl groups of 1 to 20 carbon atoms, and also alkyloxyalkylene groups of 6 to 22 carbon atoms.

The groups represented by the formula -(CH₂)ₓ-(CF₂)_{y}-R¹³ are suggested as examples of fluoroalkyl groups obtained by substituting fluorine atoms for hydrogen atoms of alkyl groups. In the formula, the index "x" is 0, 1, 2 or 3, and "y" is an integer from 1 to 20. R13 is an atom or a group chosen from a hydrogen atom, a fluorine atom,-CH(CF₃)₂- or CF(CF₃)₂. Such fluorine-substituted alkyl groups are exemplified by linear or branched polyfluoroalkyl or perfluoroalkyl groups represented by the formulae shown below:
-CF₃, -C₂F₅, -nC₃F₇, -CF(CF₃)₂, -nC₄F₉, CF₂CF(CF₃)₂, -nC₅F₁₁, -nC₆F₁₃, -nC₈F₁₇, CH₂CF₃, -(CH(CF₃)₂, CH₂CH(CF₃)₂-CH₂(CF₂)₂F, -CH₂(CF₂)₃F, -CH₂(CF₂)₄F, -CH₂(CF₂)₆F, -CH₂(CF₂)₈F, -CH₂CH₂CF₃, -CH₂CH₂(CF₂)₂F, -CH₂CH₂(CF₂)₃F, -CH₂CH₂(CF₂)₄F, -CH₂CH₂(CF₂)₆F, -CH₂CH₂(CF₂)₈F, -CH₂CH₂(CF₂)₁₀F, -CH₂CH₂(CF₂)₁₂F, -CH₂CH₂(CF₂)₁₄F, -CH₂CH₂(CF₂)₁₆F, -CH₂CH₂CH₂CF₃, -CH₂CH₂CH₂(CF₂)₂F, -CH₂CH₂CH₂(CF₂)₂H, -CH₂(CF₂)₄H and -CH₂CH₂(CF₂)₃H.

The groups represented by -CH₂CH₂-(CF₂)ₘ-CFR¹⁴-[OCF₂CF(CF₃)]ₙ-OC₃F₇ are suggested as fluoroalkyloxyfluoroalkylene groups obtained by substituting fluorine atoms for hydrogen atoms of alkyloxyalkylene groups. In the formula, the index "m" is 0 or 1, "n" is 0, 1, 2, 3, 4 or 5, and R¹⁴ is a fluorine atom or CF₃. Such fluoroalkyloxyfluoroalkylene groups are exemplified by the perfluoroalkyloxyfluoroalkylene groups represented by the formulae shown below:-CH₂CH₂CF(CF₃)-[OCF₂CF(CF₃)]n-OC₃F₇, -CH₂CH₂CF₂CF₂-[OCF₂CF(CF₃)]ₙ-OC₃F₇.

The number-average molecular weight of the vinyl polymer used in the present invention may be between 3000 and 2 000 000 and more preferably between 5000 and 800 000.

A fluorinated vinyl polymer can be one of the polymers described in the examples of Application WO 03/045337.

According to one preferred embodiment, a vinyl polymer grafted in the sense of the present invention may be conveyed in an oil or a mixture of oils, which are preferably volatile, chosen in particular from volatile silicone oils and volatile hydrocarbon-based oils, and mixtures thereof.

According to a particular embodiment, a silicone oil that is suitable for use in the invention may be cyclopentasiloxane.

According to another particular embodiment, a hydrocarbon-based oil that is suitable for use in the invention may be isododecane.

Vinyl polymers grafted with at least one unit derived from carbosiloxane dendrimer which can be particularly suitable for the present invention are the polymers sold under the names TIB 4-100, TIB 4-101, TIB 4-120, TIB 4-130, TIB 4-200, FA 4002 ID (TIB 4-202), TIB 4-220 and FA 4001 CM (TIB 4-230) by Dow Corning.

According to one particular form of the invention, the silicone polymer(s) are chosen from:
- a silicone resin of MQ type of trimethylsiloxysilicate type;
- a resin of MQ type of phenylalkylsiloxysilicate type;
- a polypropylsilsesquioxane or t-propyl silsesquioxane resin (INCI name: Polypropylsilsesquioxane (and) Isododecane);
- a vinyl polymer grafted with at least one carbosiloxane dendrimer-based unit (INCI name: Acrylates / Polytrimethylsiloxy methacrylate).

According to one particular form of the invention, the silicone polymer is a polypropylsilsesquioxane or t-propyl silsesquioxane resin (INCI name: Polypropylsilsesquioxane (and) Isododecane).

Preferably, the composition according to the invention comprises at least one silicone polymer in a content, expressed as active material of polymer, of from 0.1% to 4% by weight, preferably from 0.2% to 3% by weight, relative to the weight of the composition.

### ADDITIVES

The compositions according to the invention may also comprise additional cosmetic ingredients conventionally used in compositions intended for lip treatment. By way of examples, mention may particularly be made of active ingredients, for instance plant extracts which are preferably soluble in the composition, non-volatile, phenyl or non-phenyl silicone oils, which do not result in a single-phase mixture with the non-volatile oil or the mixture of non-volatile oils previously mentioned (conditions described in detail above), preservatives, antioxidants, sweeteners, fragrances, sunscreens, bactericides, liposoluble dyes, and also mixtures thereof.

Needless to say, those skilled in the art will take care to select the optional additional ingredients and/or the amount thereof such that the advantageous properties of the composition according to the invention are not, or are not substantially, adversely affected by the envisioned addition.

By way of illustration, the additive contents are those conventionally used in the field under consideration, and for example range from 0.01% to 5% by weight relative to the weight of the composition.

It should be noted that, if one of the additives is a non-volatile oil, or several of them, then the total content of non-volatile oils present in the composition advantageously remains within the ranges previously given in detail.

The process according to the invention consists in applying the composition previously given in detail to the lips.

The composition according to the invention may be conditioned in any type of device that is common for fluid cosmetic compositions intended in particular to be applied to the lips.

It will thus be possible to envisage devices containing a receptacle comprising an applicator fitted with a roll-on device, a receptacle comprising an applicator that can be brought into contact with the composition.

Preferably, the device comprises a reservoir fitted with stirring means, such as for example balls.

The examples below are presented as non-limiting illustrations of the field of the invention.

### EXAMPLES

### Example 1

The composition, the ingredients of which are collated in the table below, is prepared. Unless otherwise indicated, the contents are expressed in % by weight of starting material:

| **ingredients** | **Amount (%)** | |
|---|---|---|
| | **Composition according to the invention** | **Comparative composition** |
| Tridecyl trimellitate | 10 | 10 |
| Triester of a long-chain branched fatty acid and glycerol (DUB TGI 24, from Stearinerie Dubois) | 9 | 9 |
| Distearyldimethylammonium-modified hectorite (BENTONE 38 VCG from Elementis) | 0.9 | 0.9 |
| Oils/oily extracts derived from plants | 0.04 | 0.04 |
| Non-denatured 96° ethyl alcohol | 3 | 3 |
| 2-Octyldodecyl neopentanoate | 5.72 | 5.72 |
| Propylene carbonate | 0.3 | 0.3 |
| Pigments | 0.79 | 0.79 |
| T-Polypropylsilsesquioxane resin (DOW CORNING 680 ID FLUID, from Dow Corning; in solution in isododécane, 72% by weight of active material) | 2 | 2 |
| Diisostearyl malate | 21.01 | 21.01 |
| Dibutylpentaerithrityl tetrahydroxycinnamate | 0.2 | 0.2 |
| PEG-10 dimethicone (KF-6017 Shin Etsu) | 1.5 | 0.4 |
| Mica (MICA CONCORD GRADE 1000 from SCIAMA) | 2 | 2 |
| Hydrogenated polyisobutene (PARLEAM V from NOF Corporation) | 20 | 20 |
| Dodecamethylpentasiloxane, 2 cSt | 4.5 | 4.5 |
| Fragrance | 0.1 | 0.1 |
| Pentaerythrityl tetraisostearate | qs 100 | qs 100 |

The composition is prepared according to the following protocol:
The hectorite, the dibutylpentaerithrityl tetrahydroxycinnamate and the propylene glycol are mixed in a portion of the diisostearyl malate, at 90°C and homogenized in a Rayneri mixer (550 rpm). The mixture thus obtained is left to return to ambient temperature.

The oils are mixed at ambient temperature by means of a Rayneri mixer at 550 rpm.

The mixture containing the hectorite, the T-polypropylsilsesquioxane resin and the PEG-10 dimethicone is then added to the resulting mixture of oils.

After homogenization of the whole mixture in a Rayneri mixer (550 rpm), the pigments and the mica are added and the resulting mixture is again homogenized under the same conditions.

Finally, the alcohol, the fragrance and the active agents (oils/oily extracts derived from plants) are added and the whole mixture is again homogenized for 10 to 15 minutes in a Rayneri mixer (550 rpm).

In the two cases, a uniform fluid composition is obtained.

The viscosity of the composition according to the invention is 0.36 Pa.s and that of the comparative composition is 0.39 Pa.s (measured according to the protocol described in detail in the description).

The deposit for each composition is glossy, coloured, uniform, non-tacky and comfortable.

### Evaluation of the sedimentation

4.5 g of each composition are placed in a cylindrical polyethylene terephthalate receptacle, the height of which is 46.9 mm and the radius of which is 15 mm, comprising two steel balls 6.35 mm in diameter, and the whole assembly is stoppered.

The height of composition in each receptacle reaches 3.5 cm.

The two flasks are then left to stand for 48 hours at a temperature of 25°C.

After the 48 hours, the height of supernatant liquid in each of the flasks is measured.

The comparative composition did not sediment.

In the case of the composition according to the invention, a supernatant liquid representing 34.3% of the total height of composition is obtained. The appearance of the sedimentary phase is uniform, without lumps.

The product is easily re-dispersed by simple manual shaking. The composition returns to its initial uniform appearance.

The composition is applied by means of a device comprising a dip applicator of gloss type.

No difference is observed in the application of the composition directly after it has been prepared or once it has been re-dispersed after the 48 hours of storage.

The deposit is glossy, coloured, uniform, non-tacky and comfortable.

### Example 2

The composition, the ingredients of which are collated in the table below, is prepared. Unless otherwise indicated, the contents are expressed in % by weight of starting material:

| **Ingredients** | **Amount (%)** |
|---|---|
| Tridecyl trimellitate | 10 |
| Triester of a long-chain branched fatty acid and glycerol (DUB TGI 24, from Stearinerie Dubois) | 9 |
| Distearyldimethylammonium-modified hectorite (BENTONE 38 VCG from Elementis) | 0.9 |
| Oils/oily extracts derived from plants | 0.04 |
| 2-Octyldodecyl neopentanoate | 5.72 |
| Propylene carbonate | 0.3 |
| Pigments | 0.79 |
| T-Polypropylsilsesquioxane resin (DOW CORNING 680 ID FLUID, from Dow Corning; in solution in isododecane, 72% by weight of active material) | 2 |
| Diisostearyl malate | 21.01 |
| Dibutylpentaerithrityl tetrahydroxycinnamate | 0.2 |
| PEG-10 dimethicone (KF-6017 Shin Etsu) | 1.5 |
| Mica (MICA CONCORD GRADE 1000 from SCIAMA) | 2 |
| Hydrogenated polyisobutene (PARLEAM V from NOF Corporation) | 20 |
| Dodecamethylpentasiloxane, 2 cSt | 4.5 |
| Fragrance | 0.1 |
| Pentaerythrityl tetraisostearate | qs 100 |

The composition is prepared according to the protocol of Example 1, with the exception of the fact that the ethanol is no longer used.

A uniform fluid composition with a viscosity of 0.58 Pa.s (measured according to the protocol described in detail in the description) is obtained.

### Evaluation of the sedimentation

The process is performed as in Example 1.

After the 48 hours, the height of supernatant liquid is measured.

A supernatant liquid representing 28.6% of the total height of composition is obtained. The appearance of the sedimentary phase is uniform and without lumps.

The product is easily re-dispersed by simple manual shaking. The composition returns to its initial uniform appearance.

The composition is applied by means of a dip applicator of gloss type.

No difference is observed in the application of the composition directly after it has been prepared or once it has been re-dispersed after the 48 hours of storage.

The deposit is glossy, coloured, uniform, non-tacky and comfortable.

### Example 3

The composition, the ingredients of which are collated in the table below, is prepared. Unless otherwise indicated, the contents are expressed in % by weight of starting material:

| **Ingredients** | **Amount (%)** |
|---|---|
| Tridecyl trimellitate | 10 |
| Triester of a long-chain branched fatty acid and glycerol (DUB | 9 |
| TGI 24, from Stearinerie Dubois) | |
| Distearyldimethylammonium-modified hectorite (BENTONE 38 VCG from Elementis) | 0.9 |
| Oils/oily extracts derived from plants | 0.04 |
| Non-denatured 96° ethyl alcohol | 6 |
| 2-Octyldodecyl neopentanoate | 5.72 |
| Propylene carbonate | 0.3 |
| Pigments | 0.79 |
| T-Polypropylsilsesquioxane resin (DOW CORNING 680 ID FLUID, from Dow Corning; in solution in isododecane, 72% by weight of active material) | 2 |
| Diisostearyl malate | 21.01 |
| Dibutylpentaerithrityl tetrahydroxycinnamate | 0.2 |
| PEG-10 dimethicone (KF-6017 Shin Etsu) | 1.5 |
| Mica (MICA CONCORD GRADE 1000 from SCIAMA) | 2 |
| Hydrogenated polyisobutene (PARLEAM V from NOF Corporation) | 20 |
| Dodecamethylpentasiloxane, 2 cSt | 4.5 |
| Fragrance | 0.1 |
| Pentaerythrityl tetraisostearate | qs 100 |

The composition is prepared according to the protocol of Example 1.

A uniform fluid composition with a viscosity of 0.29 Pa.s (measured according to the protocol described in detail in the description) is obtained.

### Evaluation of the sedimentation

The process is performed as in Example 1.

After the 48 hours, the height of supernatant liquid is measured.

A supernatant liquid representing 48.6% of the total height of composition is obtained. The appearance of the sedimentary phase is uniform and without lumps.

The product is easily re-dispersed by simple manual shaking. The composition returns to its initial uniform appearance.

The composition is applied by means of a dip applicator of gloss type.

No difference is observed in the application of the composition directly after it has been prepared or once it has been re-dispersed after the 48 hours of storage.

The deposit is glossy, coloured, uniform, non-tacky and comfortable.

### Example 4

The composition, the ingredients of which are collated in the table below, is prepared. Unless otherwise indicated, the contents are expressed in % by weight of starting material:

| **Ingredients** | **Amount (%)** |
|---|---|
| Tridecyl trimellitate | 10 |
| Triester of a long-chain branched fatty acid and glycerol (DUB TGI 24, from Stearinerie Dubois) | 9 |
| Distearyldimethylammonium-modified hectorite (BENTONE 38 VCG from Elementis) | 1.35 |
| Oils/oily extracts derived from plants | 0.04 |
| Non-denatured 96° ethyl alcohol | 3 |
| 2-Octyldodecyl neopentanoate | 5.72 |
| Propylene carbonate | 0.45 |
| Pigments | 0.79 |
| T-Polypropylsilsesquioxane resin (DOW CORNING 680 ID FLUID, from Dow Corning; in solution in isododecane, 72% by weight of active material) | 2 |
| Diisostearyl malate | 21.01 |
| Dibutylpentaerithrityl tetrahydroxycinnamate | 0.2 |
| PEG-10 dimethicone (KF-6017 Shin Etsu) | 1.5 |
| Mica (MICA CONCORD GRADE 1000 from SCIAMA) | 2 |
| Hydrogenated polyisobutene (PARLEAM V from NOF Corporation) | 20 |
| Dodecamethylpentasiloxane, 2 cSt | 4.5 |
| Fragrance | 0.1 |
| Pentaerythrityl tetraisostearate | qs 100 |

The composition is prepared according to the protocol of Example 1.

A uniform fluid composition with a viscosity of 0.42 Pa.s (measured according to the protocol described in detail in the description) is obtained.

### Evaluation of the sedimentation

The process is performed as in Example 1.

After the 48 hours, the height of supernatant liquid is measured.

A supernatant liquid representing 48.6% of the total height of the composition is obtained. The appearance of the sedimentary phase is uniform.

The product is easily re-dispersed by simple manual shaking. The composition returns to its initial uniform appearance.

The composition is applied by means of a dip applicator of gloss type.

No difference is observed in the application of the composition directly after it has been prepared or once it has been re-dispersed after the 48 hours of storage.

The deposit is glossy, coloured, uniform, non-tacky and comfortable.

### Example 5

The composition, the ingredients of which are collated in the table below, is prepared. Unless otherwise indicated, the contents are expressed in % by weight of starting material:

| **Ingredients** | **Amount (%)** |
|---|---|
| Tridecyl trimellitate | 10 |
| Triester of a long-chain branched fatty acid and glycerol (DUB TGI 24, from Stearinerie Dubois) | 9 |
| Distearyldimethylammonium-modified hectorite (BENTONE 38 VCG from Elementis) | 0.9 |
| Oils/oily extracts derived from plants | 0.04 |
| Non-denatured 96° ethyl alcohol | 3 |
| 2-Octyldodecyl neopentanoate | 5.72 |
| Propylene carbonate | 0.3 |
| Pigments | 0.79 |
| Butyl acrylate copolymer containing dendritic silicone side chains: Tris((trimethylsiloxy)siloxyethyldimethylsiloxy)silylpropyl methacrylate in isododecane (40/60) sold under the reference Dow Corning FA 4002 | 2 |
| ID by Dow Corning | |
| Diisostearyl malate | 21.01 |
| Dibutylpentaerithrityl tetrahydroxycinnamate | 0.2 |
| PEG-10 dimethicone (KF-6017 Shin Etsu) | 1.5 |
| Mica (MICA CONCORD GRADE 1000 from SCIAMA) | 2 |
| Hydrogenated polyisobutene (PARLEAM V from NOF Corporation) | 20 |
| Dodecamethylpentasiloxane, 2 cSt | 4.5 |
| Fragrance | 0.1 |
| Pentaerythrityl tetraisostearate | qs 100 |

The composition is prepared according to the protocol of Example 1.

A uniform fluid composition with a viscosity of 0.37 Pa.s (measured according to the protocol described in detail in the description) is obtained.

### Evaluation of the sedimentation

The process is performed as in Example 1.

After the 48 hours, the height of supernatant liquid is measured.

A supernatant liquid representing 22.9% of the total height of the composition is obtained. The appearance of the sedimentary phase is uniform and without lumps.

The product is easily re-dispersed by simple manual shaking. The composition returns to its initial uniform appearance.

The composition is applied by means of a dip applicator of gloss type.

No difference is observed in the application of the composition directly after it has been prepared or once it has been re-dispersed after the 48 hours of storage.

The deposit is glossy, coloured, uniform, non-tacky and comfortable.

### Example 6

The comparative composition does not comprise any silicon surfactant but a hydrocarbon based non-ionic surfactant (polyglyceryl-4 isostearate).

The composition, the ingredients of which are collated in the table below, is prepared. Unless otherwise indicated, the contents are expressed in % by weight of starting material:

| **Ingredients** | **Amount (%)** |
|---|---|
| Tridecyl trimellitate | 10 |
| Triester of a long-chain branched fatty acid and glycerol (DUB TGI 24, from Stearinerie Dubois) | 9 |
| Distearyldimethylammonium-modified hectorite (BENTONE 38 VCG from Elementis) | 0.9 |
| Non-denatured 96° ethyl alcohol | 3 |
| 2-Octyldodecyl neopentanoate | 5.72 |
| Propylene carbonate | 0.3 |
| Pigments | 0.79 |
| T-Polypropylsilsesquioxane resin (DOW CORNING 680 ID FLUID, from Dow Corning; in solution in isododecane, 72% by weight of active material) | 1.44 |
| Diisostearyl malate | 21.01 |
| Dibutylpentaerithrityl tetrahydroxycinnamate | 0.2 |
| Polyglyceryl-4 isostearate (Isolan GI 34 from Evonik Industries) | 1.5 |
| Mica (MICA CONCORD GRADE 1000 from SCIAMA) | 2 |
| Hydrogenated polyisobutene (PARLEAM V from NOF Corporation) | 20 |
| Dodecamethylpentasiloxane, 2 cSt (XIAMETER(R) PMX-200 SILICONE FLUID 2CS, from Dow Corning) | 4.5 |
| Fragrance / sweetener | 0.1 |
| Pentaerythrityl tetraisostearate | qs 100 |

The composition is prepared according to the protocol of Example 1 and replacing the silicon surfactant by the hydrocarbon based surfactant.

A uniform fluid composition with a viscosity of 0.39 Pa.s (measured according to the protocol described in detail in the description) is obtained.

### Evaluation of the stability

The process is performed as in Example 1.

After the 48 hours, it is observed that the composition does not sediment.

### Example 7

The composition, the ingredients of which are collated in the table below, is prepared. Unless otherwise indicated, the contents are expressed in % by weight of starting material:

| **Ingredients** | **Amount (%)** |
|---|---|
| Tridecyl trimellitate | 10 |
| Triester of a long-chain branched fatty acid and glycerol (DUB TGI 24, from Stearinerie Dubois) | 9 |
| Distearyldimethylammonium-modified hectorite (BENTONE 38 VCG from Elementis) | 0,9 |
| Non-denatured 96° ethyl alcohol | 3 |
| 2-Octyldodecyl neopentanoate | 5.72 |
| Propylene carbonate | 0.45 |
| Pigments | 0.88 |
| T-Polypropylsilsesquioxane resin (DOW CORNING 680 ID FLUID, from Dow Corning; in solution in isododecane, 72% by weight of active material) | 1.44 |
| Diisostearyl malate | 21.3 |
| PEG-10 dimethicone (KF-6017 Shin Etsu) | 1 |
| Mica (MICA CONCORD GRADE 1000 from SCIAMA) | 2 |
| Hydrogenated polyisobutene (PARLEAM V from NOF Corporation) | 20 |
| Dodecamethylpentasiloxane, 2 cSt (XIAMETER(R) PMX-200 SILICONE FLUID 2CS, de Dow Corning) | 4.5 |
| Fragrance / sweetener | 0.1 |
| Pentaerythrityl tetraisostearate | qs 100 |

The composition is prepared according to the protocol of Example 1.

A uniform fluid composition with a viscosity of 0.44 Pa.s (measured according to the protocol described in detail in the description) is obtained.

### Evaluation of the sedimentation

The process is performed as in Example 1.

After the 48 hours, the height of supernatant liquid is measured.

A supernatant liquid representing 5.7% of the total height of the composition is obtained. The appearance of the sedimentary phase is uniform.

The product is easily re-dispersed by simple manual shaking. The composition returns to its initial uniform appearance.

The composition is applied by means of a dip applicator of gloss type.

No difference is observed in the application of the composition directly after it has been prepared or once it has been re-dispersed after the 48 hours of storage.

The deposit is glossy, coloured, uniform, non-tacky and comfortable.

### Example 8

The composition, the ingredients of which are collated in the table below, is prepared. Unless otherwise indicated, the contents are expressed in % by weight of starting material:

| **Ingredients** | **Amount (%)** |
|---|---|
| Tridecyl trimellitate | 10 |
| Triester of a long-chain branched fatty acid and glycerol (DUB TGI 24, from Stearinerie Dubois) | 9 |
| Distearyldimethylammonium-modified hectorite (BENTONE 38 VCG from Elementis) | 0,9 |
| Non-denatured 96° ethyl alcohol | 3 |
| 2-Octyldodecyl neopentanoate | 5.72 |
| Propylene carbonate | 0.3 |
| Pigments | 0.79 |
| T-Polypropylsilsesquioxane resin (DOW CORNING 680 ID FLUID, from Dow Corning; in solution in isododecane, 72% by weight of active material) | 1.44 |
| Diisostearyl malate | 21.01 |
| Tétrahydroxycinnamate de dibutyl pentaérithrityle | 0,2 |
| Polyglyceryl-4 isostearate (Isolan GI 34 de Evonik Industries) | 1 |
| PEG-10 dimethicone (KF-6017 Shin Etsu) | 1 |
| Mica (MICA CONCORD GRADE 1000 from SCIAMA) | 2 |
| Hydrogenated polyisobutene (PARLEAM V from NOF Corporation) | 20 |
| Dodecamethylpentasiloxane, 2 cSt (XIAMETER(R) PMX-200 SILICONE FLUID 2CS, de Dow Corning) | 4.5 |
| Fragrance / sweetener | 0.1 |
| Pentaerythrityl tetraisostearate | qs 100 |

The composition is prepared according to the protocol of Example 1.

A uniform fluid composition with a viscosity of 0.38 Pa.s (measured according to the protocol described in detail in the description) is obtained.

### Evaluation of the sedimentation

The process is performed as in Example 1.

After the 48 hours, the height of supernatant liquid is measured.

A supernatant liquid representing 11.4% of the total height of the composition is obtained. The appearance of the sedimentary phase is uniform.

The product is easily re-dispersed by simple manual shaking. The composition returns to its initial uniform appearance.

The composition is applied by means of a dip applicator of gloss type.

No difference is observed in the application of the composition directly after it has been prepared or once it has been re-dispersed after the 48 hours of storage.

The deposit is glossy, coloured, uniform, non-tacky and comfortable.

## Claims

1. Liquid cosmetic composition comprising:
* at least 70% by weight, relative to the weight of the composition, of at least one non-volatile oil or of a single-phase mixture of several non-volatile oils, the composition comprising at least one polar or non-polar, non-volatile hydrocarbon-based oil;
* at least one mineral thickener;
* at least one non-ionic silicone surfactant in a content of at least 1% by weight, relative to the weight of the composition;
* at least coloured or non-coloured solid particles.

2. Composition according to the preceding claim, **characterized in that** the non-volatile oil(s) are chosen from non-polar non-volatile hydrocarbon-based oils chosen from liquid paraffin, squalane, isoeicosane, naphthalene oil, hydrogenated or non-hydrogenated polybutenes, hydrogenated or non-hydrogenated polyisobutenes, hydrogenated or non-hydrogenated polydecenes, decene/butene copolymers, polybutene/polyisobutene copolymers, and mixtures thereof, and preferably from hydrogenated or non-hydrogenated polybutenes, hydrogenated or non-hydrogenated polyisobutenes and hydrogenated or non-hydrogenated polydecenes, and also mixtures thereof.

3. Composition according to either one of the preceding claims, **characterized in that** the non-volatile oil(s) are chosen from polar non-volatile hydrocarbon-based oils chosen from C₁₀-C₂₆ alcohols; optionally hydroxylated monoesters, diesters and triesters of a C₂-C₈ monocarboxylic or polycarboxylic acid and of a C₂-C₈ alcohol, esters of a C₂-C₈ polyol and of one or more C₂-C₈ carboxylic acids, ester oils, in particular containing between 18 and 70 carbon atoms, vinylpyrrolidone/1-hexadecene copolymers, C₁₂-C₂₆ fatty acids, which are preferably unsaturated, dialkyl carbonates, and mixtures thereof, and preferably from ester oils and in particular hydroxylated or non-hydroxylated monoesters and diesters, comprising at least 18 carbon atoms in total, triesters, in particular containing at least 35 carbon atoms, tetraesters, in particular containing at least 35 carbon atoms, and also mixtures thereof.

4. Composition according to any one of the preceding claims, **characterized in that** the content of non-volatile oil(s) represents from 70% to 90% by weight, more particularly from 75% to 90% by weight and preferably from 75% to 85% by weight, relative to the weight of the composition.

5. Composition according to any one of the preceding claims, **characterized in that** the composition optionally comprises at least one non-volatile phenyl silicone oil, preferably in a content of less than 20% by weight, relative to the weight of the composition.

6. Composition according to any one of the preceding claims, **characterized in that** the mineral thickener is chosen from optionally modified organophilic clays, silicas, which are preferably hydrophobic, silica aerogels, and also mixtures thereof, and preferably from optionally modified organophilic clays, in particular organophilic hectorites modified in particular with benzyldimethylammonium stearate chloride or with distearyldimethylammonium chloride.

7. Composition according to any one of the preceding claims, **characterized in that** the content of mineral thickener ranges from 0.2% to 2% by weight and preferably from 0.3% to 1.5% by weight, relative to the weight of the composition.

8. Composition according to any one of the preceding claims, **characterized in that** the composition comprises at least one linear or branched, preferably saturated, C₂-C₈ monoalcohol, more particularly ethanol.

9. Composition according to the preceding claim, **characterized in that** the content of C₂-C₈ monoalcohol ranges from 0% to 6% by weight and preferably from 0% to 4% by weight, relative to the weight of the composition.

10. Composition according to any one of the preceding claims, **characterized in that** the non-ionic silicone surfactant is chosen from oxyalkylenated and preferably oxyethylenated polydimethylsiloxanes, alkyl or alkoxy dimethicone copolyols, and mixtures thereof, and preferably from oxyalkylenated and preferably oxyethylenated Polydimethylsiloxanes.

11. Composition according to any one of the preceding claims, **characterized in that** the content of non-ionic silicone surfactant ranges from 1% to 3% by weight and preferably from 1% to 2% by weight, relative to the weight of the composition.

12. Composition according to any one of the preceding claims, **characterized in that** the solid particles are chosen from pigments, nacres, organic, mineral or mixed fillers, preferably mineral fillers, which are advantageously platelet-shaped, alone or as mixtures.

13. Composition according to the preceding claim, **characterized in that** the content of pigments, nacres or mixtures thereof ranges from 0.001% to 10% by weight, preferably from 0.01% to 8% by weight, relative to the weight of the composition.

14. Composition according to Claim 12, **characterized in that** the content of fillers ranges from 0.001% to 5% by weight and preferably from 0.05% to 3% by weight, relative to the weight of the composition.

15. Composition according to any one of the preceding claims, **characterized in that** it optionally comprises water, more particularly in a content not exceeding 1% by weight, more particularly not exceeding 0.5% by weight, preferably not exceeding 0.4% by weight, relative to the weight of the composition.

16. Composition according to any one of the preceding claims, **characterized in that** it comprises at least one volatile hydrocarbon-based or volatile silicone oil, or mixtures thereof.

17. Composition according to the preceding claim, **characterized in that** the content of volatile hydrocarbon-based oil(s), volatile silicone oil(s) or mixtures thereof is less than or equal to 10% by weight, more particularly less than or equal to 8% by weight, relative to the weight of the composition.

18. Composition according to any one of the preceding claims, **characterized in that** it comprises at least one silicone polymer chosen from:
- a silicone resin of MQ type of trimethylsiloxysilicate type;
- a resin of MQ type of phenylalkylsiloxysilicate type;
- a polypropylsilsesquioxane or t-propyl silsesquioxane resin,
- a vinyl polymer grafted with at least one carbosiloxane dendrimer-based unit,
and preferably a polypropylsilsesquioxane or t-propyl silsesquioxane resin.

19. Composition according to the preceding claim, **characterized in that** the content of silicone polymer, expressed as polymer active material, represents from 0.1% to 4% by weight and preferably from 0.2% to 3% by weight, relative to the weight of the composition.

20. Composition according to to any one of the preceding claims, **characterized in that** it comprises at least one additional cosmetic ingredient, more particularly chosen from active ingredients, such as plant extracts which are preferably soluble in the composition, non-volatile, phenyl or non-phenyl silicone oils, which do not result in a single-phase mixture with the non-volatile oil or the mixture of non-volatile oils previously mentioned, preservatives, antioxidants, sweeteners, fragrances, sunscreens, bactericides, liposoluble dyes, and also mixtures thereof.

21. Composition according to the preceding claim, **characterized in that** the content of additives is ranging from 0.01% to 5% by weight relative to the weight of the composition.

22. Composition according to any one of the preceding claims, **characterized in that** it comprises at least one additional hydrocarbon based surfactant, preferably non-ionic.

23. Composition according to the preceding claim, **characterized in that** the additional hydrocarbon based surfactant(s) are chosen from alkyl and polyalkyl esters of poly(ethylene oxide); oxyalkylenated alcohols; alkyl and polyalkyl ethers of poly(ethylene oxide); optionally polyoxyethylenated alkyl and polyalkyl esters of sorbitan; optionally polyoxyethylenated alkyl and polyalkyl ethers of sorbitan; alkyl and polyalkyl glycosides or polyglycosides, in particular alkyl and polyalkyl glucosides or polyglucosides; alkyl and polyalkyl esters of sucrose; optionally polyoxyethylenated alkyl and polyalkyl esters, and preferably mono-alkyl esters, of glycerol, and optionally polyoxyethylenated alkyl and polyalkyl ethers of glycerol, and mixtures thereof; more particularly the number of oxyalkylenated units (preferably oxyethylenated and/or oxypropylenated) ranging from 1 to 200; the alkyl chain length ranging from 8 to 30 carbon atoms, preferably 8 to 24 carbon atoms.

24. Composition according to any one of claim 22 or 23, **characterized in that** the amount of additional hydrocarbon based surfactant(s) is such that the total amount of surfactants is of at most 3% by weight, relative to the weight of the composition.

25. Composition according to any one of claim 22 to 24, **characterized in that** the ratio of the amount of additional(s) hydrocarbon based surfactant(s) relative to the total amount of silicon surfactant(s) and additional hydrocarbon based surfactant(s) is less than or equal to 0.7, preferably less than or equal to 0.5 and more particularly less than or equal to 0.2 and even more preferably less than 0.1.

26. Composition according to any one of claim 22 to 25, **characterized in that** it does not comprise any additional hydrocarbon based surfactant(s).

27. Composition according to any one of the preceding claims, **characterized in that** it sediments when it is left to stand, more particularly it allows the appearance of a supernatant liquid after standing for 48 hours at 25°C.

28. Composition according to any one of the preceding claims, **characterized in that** the viscosity of the composition varies between 0.2 and 0.8 Pa.s, preferably between 0.3 and 0.7 Pa.s, performed at 25°C, using a Rheomat RM180 viscometer equipped with a No. 2 spindle, performed after 10 minutes of rotation of the spindle in the composition, at 200 rpm.

29. Process for making up and/or caring for the lips, in which the composition according to any one of the preceding claims is applied.

## Patentansprüche

1. Flüssige kosmetische Zusammensetzung, umfassend:
* mindestens 70 Gew.-%, bezogen auf das Gewicht der Zusammensetzung, mindestens eines nichtflüchtigen Öls oder einer einphasigen Mischung mehrerer nichtflüchtiger Öle, wobei die Zusammensetzung mindestens ein polares oder unpolares, nichtflüchtiges Öl auf Kohlenwasserstoffbasis umfasst;
* mindestens einen mineralischen Verdicker;
* mindestens ein nichtionisches Silikontensid in einem Gehalt von mindestens 1 Gew.-%, bezogen auf das Gewicht der Zusammensetzung;
* mindestens farbige oder nichtfarbige feste Teilchen.

2. Zusammensetzung nach einem der vorhergehenden Ansprüche, **dadurch gekennzeichnet, dass** das nichtflüchtige Öl bzw. die nichtflüchtigen Öle aus unpolaren nichtflüchtigen Ölen auf Kohlenwasserstoffbasis ausgewählt ist bzw. sind, die aus Flüssigparaffin, Squalan, Isoeicosan, Naphthalin-Öl, hydrierten oder nicht hydrierten Polybutenen, hydrierten oder nicht hydrierten Polyisobutenen, hydrierten oder nicht hydrierten Polydecenen, Decen/Buten-Copolymeren, Polybuten/Polyisobuten-Copolymeren und Mischungen davon und vorzugsweise aus hydrierten oder nicht hydrierten Polybutenen, hydrierten oder nicht hydrierten Polyisobutenen und hydrierten oder nicht hydrierten Polydecenen sowie Mischungen davon ausgewählt sind.

3. Zusammensetzung nach einem der vorhergehenden Ansprüche, **dadurch gekennzeichnet, dass** das nichtflüchtige Öl bzw. die nichtflüchtigen Öle aus polaren nichtflüchtigen Ölen auf Kohlenwasserstoffbasis ausgewählt ist bzw. sind, die aus C₁₀-C₂₆-Alkoholen, gegebenenfalls hydroxylierten Monoestern, Diestern und Triestern einer C₂-C₈-Mono- oder -Polycarbonsäure und eines C₂-C₈-Alkohols, Estern eines C₂-C₈-Polyols und einer oder mehrerer C₂-C₈-Carbonsäuren, Esterölen, die insbesondere zwischen 18 und 70 Kohlenstoffatome enthalten, Vinylpyrrolidon/1-Hexadecen-Copolymeren, C₁₂-C₂₆-Fettsäuren, die vorzugsweise ungesättigt sind, Dialkylcarbonaten und Mischungen davon und vorzugsweise aus Esterölen und insbesondere hydroxylierten oder nicht hydroxylierten Monoestern und Diestern mit insgesamt mindestens 18 Kohlenstoffatomen, Triestern, die insbesondere mindestens 35 Kohlenstoffatome enthalten, Tetraestern, die insbesondere mindestens 35 Kohlenstoffatome enthalten, sowie Mischungen davon ausgewählt sind.

4. Zusammensetzung nach einem der vorhergehenden Ansprüche, **dadurch gekennzeichnet, dass** der Gehalt an nichtflüchtigem Öl bzw. nichtflüchtigen Ölen 70 bis 90 Gew.-%, spezieller 75 bis 90 Gew.-% und vorzugsweise 75 bis 85 Gew.-%, bezogen auf das Gewicht der Zusammensetzung, ausmacht.

5. Zusammensetzung nach einem der vorhergehenden Ansprüche, **dadurch gekennzeichnet, dass** die Zusammensetzung gegebenenfalls mindestens ein nichtflüchtiges Phenylsilikonöl umfasst, vorzugsweise in einem Gehalt von weniger als 20 Gew.-%, bezogen auf das Gewicht der Zusammensetzung.

6. Zusammensetzung nach einem der vorhergehenden Ansprüche, **dadurch gekennzeichnet, dass** der mineralische Verdicker aus gegebenenfalls modifizierten organophilen Tonen, Siliciumdioxiden, die vorzugsweise hydrophob sind, Siliciumdioxid-Aerogelen sowie Gemischen davon und vorzugsweise aus gegebenenfalls modifizierten organophilen Tonen, insbesondere organophilen Hectoriten, die insbesondere mit Benzyldimethylammoniumstearatchlorid oder mit Distearyldimethylammoniumchlorid modifiziert sind, ausgewählt ist.

7. Zusammensetzung nach einem der vorhergehenden Ansprüche, **dadurch gekennzeichnet, dass** der Gehalt an mineralischem Verdicker im Bereich von 0,2 bis 2 Gew.-% und vorzugsweise von 0,3 bis 1,5 Gew.-%, bezogen auf das Gewicht der Zusammensetzung, liegt.

8. Zusammensetzung nach einem der vorhergehenden Ansprüche, **dadurch gekennzeichnet, dass** die Zusammensetzung mindestens einen linearen oder verzweigten, vorzugsweise gesättigten, C₂-C₈-Monoalkohol, spezieller Ethanol, umfasst.

9. Zusammensetzung nach dem vorhergehenden Anspruch, **dadurch gekennzeichnet, dass** der Gehalt an C₂-C₈-Monoalkohol im Bereich von 0 bis 6 Gew.-% und vorzugsweise von 0 bis 4 Gew.-%, bezogen auf das Gewicht der Zusammensetzung, liegt.

10. Zusammensetzung nach einem der vorhergehenden Ansprüche, **dadurch gekennzeichnet, dass** das nicht ionische Silikontensid aus oxyalkylenierten und vorzugsweise oxyethylenierten Polydimethylsiloxanen, Alkyl- oder Alkoxydimethiconcopolyolen und Mischungen davon und vorzugsweise aus oxyalkylierten und vorzugsweise oxyethylenierten Polydimethylsiloxanen ausgewählt ist.

11. Zusammensetzung nach einem der vorhergehenden Ansprüche, **dadurch gekennzeichnet, dass** der Gehalt an nichtionischem Silikontensid im Bereich von 1 bis 3 Gew.-% und vorzugsweise von 1 bis 2 Gew.-%, bezogen auf das Gewicht der Zusammensetzung, liegt.

12. Zusammensetzung nach einem der vorhergehenden Ansprüche, **dadurch gekennzeichnet, dass** die festen Teilchen aus Pigmenten, Perlglanzmitteln, organischen, mineralischen oder gemischten Füllstoffen, vorzugsweise mineralischen Füllstoffen, die vorzugsweise plättchenförmig sind, allein oder als Mischungen ausgewählt sind.

13. Zusammensetzung nach dem vorhergehenden Anspruch, **dadurch gekennzeichnet, dass** der Gehalt an Pigmenten, Perlglanzmitteln oder Mischungen davon im Bereich von 0,001 bis 10 Gew.-%, vorzugsweise von 0,01 bis 8 Gew.-%, bezogen auf das Gewicht der Zusammensetzung, liegt.

14. Zusammensetzung nach Anspruch 12, **dadurch gekennzeichnet, dass** der Gehalt an Füllstoffen im Bereich von 0,001 bis 5 Gew.-% und vorzugsweise von 0,05 bis 3 Gew.-%, bezogen auf das Gewicht der Zusammensetzung, liegt.

15. Zusammensetzung nach einem der vorhergehenden Ansprüche, **dadurch gekennzeichnet, dass** sie gegebenenfalls Wasser enthält, spezieller in einem Gehalt von höchstens 1 Gew.-%, spezieller höchstens 0,5 Gew.-%, vorzugsweise höchstens 0,4 Gew.-%, bezogen auf das Gewicht der Zusammensetzung.

16. Zusammensetzung nach einem der vorhergehenden Ansprüche, **dadurch gekennzeichnet, dass** sie mindestens ein flüchtiges Öl auf Kohlenwasserstoffbasis oder flüchtiges Silikonöl oder Mischungen davon umfasst.

17. Zusammensetzung nach dem vorhergehenden Anspruch, **dadurch gekennzeichnet, dass** der Gehalt an flüchtigem Öl auf Kohlenwasserstoffbasis bzw. flüchtigen Ölen auf Kohlenwasserstoffbasis, flüchtigem Silikonöl bzw. flüchtigen Silikonölen oder Mischungen davon kleiner oder gleich 10 Gew.-%, spezieller kleiner oder gleich 8 Gew.-%, bezogen auf das Gewicht der Zusammensetzung, ist.

18. Zusammensetzung nach einem der vorhergehenden Ansprüche, **dadurch gekennzeichnet, dass** sie mindestens ein Silikonpolymer umfasst, das aus
- einem Silikonharz vom MQ-Typ vom Trimethylsiloxysilikat-Typ;
- einem Harz vom MQ-Typ von Phenylalkylsiloxysilikat-Typ:
- einem Polypropylsilsesquioxan- oder t-Propylsilsesquioxanharz;
- einem Vinylpolymer, das mit mindestens einer auf Carbosiloxandendrimer basierenden Einheit gepfropft ist,
und vorzugsweise einem Polypropylsilsesquioxan- oder t-Propylsilsesquioxanharz ausgewählt ist.

19. Zusammensetzung nach dem vorhergehenden Anspruch, **dadurch gekennzeichnet, dass** der Gehalt an Silikonpolymer, ausgedrückt als Polymer-Aktivsubstanz, 0,1 bis 4 Gew.-% und vorzugsweise 0,2 bis 3 Gew.-%, bezogen auf das Gewicht der Zusammensetzung, ausmacht.

20. Zusammensetzung nach einem der vorhergehenden Ansprüche, **dadurch gekennzeichnet, dass** sie mindestens einen zusätzlichen kosmetischen Bestandteil umfasst, der spezieller aus Wirkstoffen, wie Pflanzenextrakten, die vorzugsweise in der Zusammensetzung löslich sind, nichtflüchtigen Phenyl- oder Nichtphenylsilikonölen, die nicht zu einer einphasigen Mischung mit dem nichtflüchtigen Öl oder der Mischung von nichtflüchtigen Ölen gemäß obigen Angaben führen, Konservierungsstoffen, Antioxidantien, Süßungsmitteln, Duftstoffen, Lichtschutzmitteln, Bakteriziden, fettlöslichen Farbstoffen sowie Mischungen davon ausgewählt ist.

21. Zusammensetzung nach dem vorhergehenden Anspruch, **dadurch gekennzeichnet, dass** der Gehalt an Additiven im Bereich von 0,01 bis 5 Gew.-%, bezogen auf das Gewicht der Zusammensetzung, liegt.

22. Zusammensetzung nach einem der vorhergehenden Ansprüche, **dadurch gekennzeichnet, dass** sie mindestens ein zusätzliches Tensid auf Kohlenwasserstoffbasis umfasst, das vorzugsweise nichtionisch ist.

23. Zusammensetzung nach dem vorhergehenden Anspruch, **dadurch gekennzeichnet, dass** das zusätzliche Tensid auf Kohlenwasserstoffbasis bzw. die zusätzlichen Tenside auf Kohlenwasserstoffbasis aus Alkyl- und Polyalkylestern von Poly-(ethylenoxid); oxyalkylenierten Alkoholen; Alkyl- und Polyalkylethern von Poly(ethylenoxid); gegebenenfalls polyoxyethylenierten Alkyl- und Polyalkylestern von Sorbitan; gegebenenfalls polyoxyethylenierten Alkyl- und Polyalkylethern von Sorbitan; Alkyl- und Polyalkylglycosiden oder -polyglycosiden, insbesondere Alkyl- und Polyalkylglucosiden oder -polyglucosiden; Alkyl- und Polyalkylestern von Saccharose; gegebenenfalls polyoxyethylenierten Alkyl- und Polyalkylestern und vorzugsweise Monoalkylestern von Glycerin und gegebenenfalls polyoxyethylenierten Alkyl- und Polyalkylethern von Glycerin und Mischungen davon ausgewählt ist bzw. sind; spezieller die Zahl von oxyalkylenierten (vorzugsweise oxyethylenierten und/oder oxypropylenierten) Einheiten im Bereich von 1 bis 200 liegt; wobei die Alkylkettenlänge im Bereich von 8 bis 30 Kohlenstoffatomen und vorzugsweise 8 bis 24 Kohlenstoffatomen liegt.

24. Zusammensetzung nach Anspruch 22 oder 23, **dadurch gekennzeichnet, dass** die Menge an zusätzlichem Tensid auf Kohlenwasserstoffbasis bzw. zusätzlichen Tensiden auf Kohlenwasserstoffbasis so beschaffen ist, dass die Gesamtmenge an Tensiden höchstens 3 Gew.-%, bezogen auf das Gewicht der Zusammensetzung, beträgt.

25. Zusammensetzung nach einem der Ansprüche 22 bis 24, **dadurch gekennzeichnet, dass** das Verhältnis der Menge an zusätzlichem Tensid auf Kohlenwasserstoffbasis bzw. zusätzlichen Tensiden auf Kohlenwasserstoffbasis zur Gesamtmenge von Silikontensid bzw. Silikontensiden und zusätzlichem Tensid auf Kohlenwasserstoffbasis bzw. zusätzlichen Tensiden auf Kohlenwasserstoffbasis kleiner oder gleich 0,7, vorzugsweise kleiner oder gleich 0,5 und spezieller kleiner oder gleich 0,2 und noch weiter bevorzugt kleiner 0,1 ist.

26. Zusammensetzung nach einem der Ansprüche 22 bis 25, **dadurch gekennzeichnet, dass** sie keine zusätzlichen Tenside auf Kohlenwasserstoffbasis umfasst.

27. Zusammensetzung nach einem der vorhergehenden Ansprüche, **dadurch gekennzeichnet, dass** sie beim Stehenlassen sedimentiert und spezieller nach 48 Stunden Stehen bei 25 °C das Erscheinen einer überstehenden Flüssigkeit ermöglicht.

28. Zusammensetzung nach einem der vorhergehenden Ansprüche, **dadurch gekennzeichnet, dass** die Viskosität der Zusammensetzung zwischen 0,2 und 0,8 Pa.s, vorzugsweise zwischen 0,3 und 0,7 Pa.s, variiert, durchgeführt bei 25 °C unter Verwendung eines Viskosimeters der Bauart Rheomat RM180 mit einer Spindel Nr. 2, durchgeführt nach 10 Minuten Drehung der Spindel in der Zusammensetzung mit 200 U/min.

29. Verfahren zum Schminken und/oder Pflegen der Lippen, bei dem man die Zusammensetzung nach einem der vorhergehenden Ansprüche aufbringt.

## Revendications

1. Composition cosmétique liquide, comprenant :
* au moins 70 % en poids, par rapport au poids de la composition, d'au moins une huile non volatile ou d'un mélange monophasique de plusieurs huiles non volatiles, la composition comprenant au moins une huile hydrocarbonée non volatile, polaire ou non polaire ;
* au moins un épaississant minéral ;
* au moins un agent tensioactif siliconé non ionique selon une teneur d'au moins 1 % en poids, par rapport au poids de la composition ;
* au moins des particules solides, colorées ou non colorées.

2. Composition selon la revendication précédente, **caractérisée en ce que** la ou les huiles non volatiles sont choisies parmi les huiles hydrocarbonées non volatiles et non polaires choisies parmi l'huile de paraffine, le squalane, l'isoéicosane, l'huile de naphtalène, les polybutènes hydrogénés ou non hydrogénés, les polyisobutènes hydrogénés ou non hydrogénés, les polydécènes hydrogénés ou non hydrogénés, les copolymères de décène/butène, les copolymères de polybutène/ polyisobutène, et des mélanges de ceux-ci ; et de préférence parmi les polybutènes hydrogénés ou non hydrogénés, les polyisobutènes hydrogénés ou non hydrogénés, les polydécènes hydrogénés ou non hydrogénés, ainsi que des mélanges de ceux-ci.

3. Composition selon l'une ou l'autre parmi les revendications précédentes, **caractérisée en ce que** la ou les huiles non volatiles sont choisies parmi les huiles hydrocarbonées non volatiles polaires choisies parmi les C₁₀-C₂₆-alcools ; les monoesters, les diesters, les triesters, optionnellement hydroxylés, d'un acide mono- ou polycarboxylique en C₂-C₈ et d'un C₂-C₈-alcool, les esters d'un C₂-C₈-polyol et d'un ou plusieurs acides C₂-C₈-carboxyliques, les huiles ester, en particulier contenant entre 18 et 70 atomes de carbone, les copolymères de vinylpyrrolidone/1-hexadécène, les acides gras en C₁₂-C₂₆, qui sont de préférence insaturés, les carbonates de dialkyle, et des mélanges de ceux-ci, et de préférence parmi les huiles esters, et en particulier les monoesters, les diesters, hydroxylés ou non hydroxylés, comprenant au moins 18 atomes de carbone au total, les triesters, notamment contenant au moins 35 atomes de carbone, les tétraesters, notamment contenant au moins 35 atomes de carbone, ainsi que des mélanges de ceux-ci.

4. Composition selon l'une quelconque des revendications précédentes, **caractérisée en ce que** la teneur en huile(s) non volatile(s) représente de 70 % à 90 % en poids, plus particulièrement de 75 % à 90 % en poids, et de préférence de 75 % à 85 % en poids, par rapport au poids de la composition.

5. Composition selon l'une quelconque des revendications précédentes, **caractérisée en ce que** la composition comprend éventuellement au moins une huile siliconée phénylée non volatile, de préférence selon une teneur inférieure à 20 % en poids, par rapport au poids de la composition.

6. Composition selon l'une quelconque des revendications précédentes, **caractérisée en ce que** l'épaississant minéral est choisi parmi les argiles organophiles éventuellement modifiées, les silices, qui sont de préférences hydrophobes, les aérogels de silice, ainsi que des mélanges de ceux-ci, et de préférence parmi les argiles organophiles éventuellement modifiées, en particulier les hectorites organophiles modifiées notamment par le chlorure de stéarate de benzyldiméthyl-ammonium, ou par le chlorure de distéaryl diméthylammonium.

7. Composition selon l'une quelconque des revendications précédentes, **caractérisée en ce que** la teneur en épaississant minéral va de 0,2 % à 2 % en poids, et de préférence de 0,3 % à 1,5 % en poids, par rapport au poids de la composition.

8. Composition selon l'une quelconque des revendications précédentes, **caractérisée en ce que** la composition comprend au moins un mono-alcool en C₂-C₈, linéaire ou ramifié, de préférence saturé, plus particulièrement l'éthanol.

9. Composition selon la revendication précédente, **caractérisée en ce que** la teneur en mono-alcool en C₂-C₈ va de 0 % à 6 % en poids, et de préférence de 0 % à 4 % en poids, par rapport au poids de la composition.

10. Composition selon l'une quelconque des revendications précédentes, **caractérisée en ce que** l'agent tensioactif siliconé non ionique est choisi parmi les polydiméthylsiloxanes oxyalkylénés et de préférence oxyéthylénés, les alkyl- ou alcoxy-diméthicone copolyols, ainsi que des mélanges de ceux-ci, et de préférence parmi les polydiméthylsiloxanes oxyalkylénés et de préférence oxyéthylénés.

11. Composition selon l'une quelconque des revendications précédentes, **caractérisée en ce que** la teneur en agent tensioactif siliconé non ionique va de 1 % à 3 % en poids, et de préférence de 1 % à 2 % en poids, par rapport au poids de la composition.

12. Composition selon l'une quelconque des revendications précédentes, **caractérisée en ce que** les particules solides sont choisies parmi les pigments, les nacres, les charges organiques, minérales ou mixtes, de préférence les charges minérales, qui sont de manière avantageuse plaquettaires, seuls ou sous forme de mélanges.

13. Composition selon la revendication précédente, **caractérisée en ce que** la teneur en pigments, nacres ou mélanges de ceux-ci, va de 0,001 % à 10 % en poids, de préférence de 0,01 % à 8 % en poids, par rapport au poids de la composition.

14. Composition selon la revendication 12, **caractérisée en ce que** la teneur en charges va de 0,001 % à 5 % en poids, et de préférence de 0,05 % à 3 % en poids, par rapport au poids de la composition.

15. Composition selon l'une quelconque des revendications précédentes, **caractérisée en ce qu'**elle comprend éventuellement de l'eau, plus particulièrement selon une teneur ne dépassant pas 1 % en poids, plus particulièrement ne dépassant pas 0,5 % en poids, de préférence ne dépassant pas 0,4 % en poids, par rapport au poids de la composition.

16. Composition selon l'une quelconque des revendications précédentes, **caractérisée en ce qu'**elle comprend au moins une huile hydrocarbonée volatile ou siliconée volatile, ou des mélanges de celles-ci.

17. Composition selon la revendication précédente, **caractérisée en ce que** la teneur en huile(s) hydrocarbonée (s) volatile (s), en huile (s) siliconée (s) volatile(s) ou en mélanges de celles-ci, est inférieure ou égale à 10 % en poids, plus particulièrement inférieure ou égale à 8 % en poids, par rapport au poids de la composition.

18. Composition selon l'une quelconque des revendications précédentes, **caractérisée en ce qu'**elle comprend au moins un polymère siliconé choisi parmi :
- une résine siliconée de type MQ de type triméthylsiloxysilicate ;
- une résine de type MQ de type phénylalkylsiloxysilicate ;
- une résine de polypropylsilsesquioxane ou de t-propyl silsesquioxane ;
- un polymère vinylique greffé avec au moins un motif dérivé de dendrimère carbosiloxane ;
et de préférence une résine de polypropylsilsesquioxane ou de t-propyl silsesquioxane.

19. Composition selon la revendication précédente, **caractérisée en ce que** la teneur en polymère siliconé, exprimée en matière active de polymère, représente de 0,1 % à 4 % en poids, et de préférence de 0,2 % à 3 % en poids, par rapport au poids de la composition.

20. Composition selon l'une quelconque des revendications précédentes, **caractérisée en ce qu'**elle comprend au moins un ingrédient cosmétique supplémentaire, choisi plus particulièrement parmi les ingrédients actifs tels que les extraits de plantes qui sont de préférence solubles dans la composition, les huiles siliconées non volatiles phénylées ou non phénylées, qui ne conduisent pas à un mélange monophasique avec l'huile non volatile ou le mélange d'huiles non volatiles précédemment mentionné, les conservateurs, les antioxydants, les édulcorants, les fragrances, les écrans solaires, les bactéricides, les colorants liposolubles, ainsi que des mélanges de ceux-ci.

21. Composition selon la revendication précédente, **caractérisée en ce que** la teneur en additifs va de 0,01 % à 5 % en poids par rapport au poids de la composition.

22. Composition selon l'une quelconque des revendications précédentes, **caractérisée en ce qu'**elle comprend au moins un agent tensioactif hydrocarboné supplémentaire, de préférence non ionique.

23. Composition selon la revendication précédente, **caractérisée en ce que** le ou les agents tensioactifs hydrocarbonés supplémentaires sont choisis parmi les alkyl- et polyalkylesters de poly(oxyde d'éthylène) ; les alcools oxyalkylénés ; les alkyl- et polyalkyléthers de poly(oxyde d'éthylène) ; les alkyl- et polyalkylesters de sorbitane éventuellement polyoxyéthylénés ; les alkyl- et polyalkyléthers de sorbitane éventuellement polyoxyéthylénés ; les alkyl- et polyalkyl-glycosides ou polyglycosides, en particulier les alkyl- et polyalkyl-glucosides ou polyglucosides ; les alkyl- et polyalkylesters de saccharose ; les alkyl- et polyalkylesters et de préférence monoalkylesters, éventuellement polyoxyéthylénés, de glycérol, et les alkyl- et polyalkyléthers éventuellement polyoxyéthylénés de glycérol, ainsi que des mélanges de ceux-ci ; plus particulièrement le nombre de motifs oxyalkylénés (de préférence oxyéthylénés et/ou oxypropylénés) allant de 1 à 200 ; la longueur de chaîne alkyle allant de 8 à 30 atomes de carbone, de préférence de 8 à 24 atomes de carbone.

24. Composition selon l'une quelconque des revendications 22 ou 23, **caractérisée en ce que** la quantité d'agent(s) tensioactif(s) hydrocarboné(s) supplémentaire(s) est telle que la quantité totale d'agents tensioactifs est d'au plus 3 % en poids, par rapport au poids de la composition.

25. Composition selon l'une quelconque des revendications 22 à 24, **caractérisée en ce que** le rapport de la quantité d'agent(s) tensioactif(s) hydrocarboné(s) supplémentaire(s) par rapport à la quantité totale d'agent (s) tensioactif (s) siliconé(s) et d'agent (s) tensioactif(s) hydrocarboné(s) supplémentaire(s) est inférieur ou égal à 0,7, de préférence inférieur ou égal à 0,5, et plus particulièrement inférieur ou égal à 0,2 et encore plus préférablement inférieur à 0,1.

26. Composition selon l'une quelconque des revendications 22 à 25, **caractérisée en ce qu'**elle ne comprend aucun agent tensioactif hydrocarboné supplémentaire.

27. Composition selon l'une quelconque des revendications précédentes, **caractérisée en ce qu'**elle sédimente lorsqu'on la laisse reposer, plus particulièrement elle permet l'apparition d'un surnageant liquide après repos pendant 48 heures à 25°C.

28. Composition selon l'une quelconque des revendications précédentes, **caractérisée en ce que** la viscosité de la composition est comprise entre 0,2 et 0,8 Pa.s, de préférence entre 0,3 et 0,7 Pa.s, mesurée à 25°C à l'aide d'un viscosimètre Rheomat RM180 équipé d'une tige n°2, mesurée après 10 minutes de rotation de la tige dans la composition à 200 tours/min.

29. Procédé de maquillage et/ou soin des lèvres, dans lequel on applique la composition selon l'une quelconque des revendications précédentes.
